# EUROPÄISCHE PATENTSCHRIFT

(11) **EP 0 802 904 B1**
(45) Veröffentlichungstag und Bekanntmachung des Hinweises auf die Patenterteilung: **09.09.1998**
(21) Anmeldenummer: 96900563.6
(22) Anmeldetag: 04.01.1996
(51) Int. Cl.: C07D 213/61, A01N 43/40

(54) **SUBSTITUIERTE 2-PHENYLPYRIDINE ALS HERBIZIDE**
SUBSTITUTED 2-PHENYLPYRIDINES AS HERBICIDES
2-PHENYLPYRIDINES SUBSTITUEES UTILISEES COMME HERBICIDES

(30) Priorität: 13.01.1995 DE 19500760
(43) Veröffentlichungstag der Anmeldung: 29.10.1997
(73) Patentinhaber: BASF AKTIENGESELLSCHAFT, 67056 Ludwigshafen (DE)
(72) Erfinder: SCHÄFER, Peter, D-67308 Ottersheim (DE); HAMPRECHT, Gerhard, D-69469 Weinheim (DE); HEISTRACHER, Elisabeth, D-67061 Ludwigshafen (DE); KLINTZ, Ralf, D-67269 Grünstadt (DE); KÖNIG, Hartmann, D-69115 Heidelberg (DE); WALTER, Helmut, D-67283 Obrigheim (DE); WESTPHALEN, Karl-Otto, D-67346 Speyer (DE); MISSLITZ, Ulf, D-67433 Neustadt (DE)
(86) Internationale Anmeldenummer: EP9600022
(87) Internationale Veröffentlichungsnummer: WO9621647

(56) Entgegenhaltungen:
- EP-A- 0 463 492
- DE-A- 4 323 916
- CHEMICAL ABSTRACTS, vol. 114, no. 11, 18.März 1991 Columbus, Ohio, US; abstract no. 96724k, N.P. MEL'NIKOVA ET AL. 'Photodegradation of the herbicide Zellek.' Seite 243; in der Anmeldung erwähnt & IZVESTIYA TIMIRYAZEVSKOI SEL'SKOKHOZYAISTVENNOI AKADEMII, Nr. 3, 1990 MOSCOW, Seiten 155-160,

## Beschreibung

Die vorliegende Erfindung betrifft neue substituierte 2-Phenylpyridine der Formel I 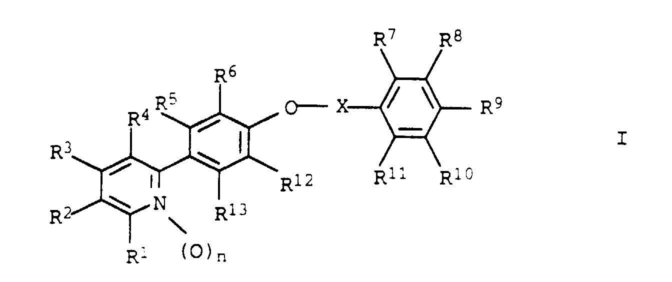 in der die Variablen folgende Bedeutung haben:
- n: 0 oder 1;
- R¹, R³, R⁴: unabhängig voneinander Wasserstoff, Halogen, C₁-C₄-Alkyl, C₁-C₄-Halogenalkyl, Hydroxy, C₁-C₄-Alkoxy, C₁-C₄-Halogenalkoxy, Nitro, Amino, C₁-C₄-Alkylamino, Di-(C₁-C₄-alkyl)-amino, Mercapto, C₁-C₄-Alkylthio, C₁-C₄-Halogenalkylthio, Cyano, Hydroxycarbonyl, (C₁-C₄-Alkoxy)carbonyl, Aminocarbonyl, C₁-C₄-Alkylaminocarbonyl oder Di- (C₁-C₄-alkyl)-aminocarbonyl;
- R²: Wasserstoff, Halogen, C₁-C₄-Alkyl, C₁-C₄-Halogenalkyl, Cyano, Nitro, Amino, Hydroxy, C₁-C₄-Halogenalkoxy, Mercapto, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl oder C₁-C₄-Halogenalkylsulfonyl;
- R⁵: Wasserstoff, Halogen oder Cyano;
- R⁶ und R¹³: unabhängig voneinander Wasserstoff oder Halogen;
- X: Methylen oder Carbonyl;
- R⁷, R⁸, R⁹, R¹⁰: unabhängig voneinander Wasserstoff, Nitro, Cyano, Halogen, C₁-C₄-Alkyl, C₁-C₄-Halogenalkyl, C₁-C₄-Alkoxy oder C₁-C₄-Halogenalkoxy;
- R¹¹: Wasserstoff, Halogen, C₁-C₄-Alkyl, C₁-C₄-Halogenalkyl, C₁-C₄-Hydroxyalkyl, C₁-C₄-Alkoxy-C₁-C₄-alkyl, C₃-C₆-Cycloalkyl, C₂-C₄-Alkenyl, C₂-C₄-Alkinyl, Hydroxycarbonyl-C₁-C₄-alkyl, Hydroxycarbonyl-C₂-C₄-alkenyl, Hydroxycarbonyl-C₂-C₄-alkinyl, (C₁-C₄-Alkoxy)carbonyl-C₁-C₄-alkyl, (C₁-C₄-Alkoxy)carbonyl-C₂-C₄-alkenyl, (C₁-C₄-Alkoxy)carbonyl-C₂-C₄-alkinyl, (C₁-C₄-Alkoxy)carbonyl-C₂-C₄-halogenalkyl, (C₁-C₄-Alkoxy)carbonyl-C₂-C₄-halogenalkenyl, Aminocarbonyl-C₁-C₄-alkyl, Aminocarbonyl-C₂-C₄-alkenyl, Aminocarbonyl-C₂-C₄-alkinyl, C₁-C₄-Alkylaminocarbonyl-C₁-C₄-alkyl, C₁-C₄-Alkylaminocarbonyl-C₂-C₄-alkenyl, C₁-C₄-Alkylaminocarbonyl-C₂-C₄-alkinyl, Di-(C₁-C₄-alkyl)-aminocarbonyl-C₁-C₄-alkyl, Di-(C₁-C₄-alkyl)-aminocarbonyl-C₂-C₄-alkenyl, Di-(C₁-C₄-alkyl)-aminocarbonyl-C₂-C₄-alkinyl, Aminocarbonyl-C₁-C₄-halogenalkyl, Aminocarbonyl-C₂-C₄-halogenalkenyl, C₁-C₄-Alkylaminocarbonyl-C₁-C₄-halogenalkyl, C₁-C₄-Alkylaminocarbonyl-C₂-C₄-halogenalkenyl, Di-(C₁-C₄-alkyl)-aminocarbonyl-C₂-C₄-halogenalkyl, Di-(C₁-C₄-alkyl)-aminocarbonyl-C₂-C₄-halogenalkenyl, (Hydroxycarbonyl-C₁-C₄-alkyl)-aminocarbonyl-C₂-C₄-alkenyl, (Hydroxycarbonyl-C₁-C₄-alkyl)-aminocarbonyl-C₂-C₄-halogenalkenyl, N-(Hydroxycarbonyl-C₁-C₄-alkyl)-N-(C₁-C₄-alkyl)-aminocarbonyl-C₂-C₄-alkenyl, N-(Hydroxycarbonyl-C₁-C₄-alkyl)-N-(C₁-C₄-alkyl)-aminocarbonyl-C₂-C₄-halogenalkenyl, [(C₁-C₄-Alkoxy)carbonyl-C₁-C₄-alkyl]-aminocarbonyl-C₂-C₄-alkenyl, [(C₁-C₄-Alkoxy)carbonyl-C₁-C₄-alkyl]-aminocarbonyl-C₂-C₄-halogenalkenyl, N-[(C₁-C₄-Alkoxy)carbonyl-C₁-C₄-alkyl]-N-(C₁-C₄-alkyl)-aminocarbonyl-C₂-C₄-alkenyl, N-[(C₁-C₄-Alkoxy)carbonyl-C₁-C₄-alkyl]-N-(C₁-C₄-alkyl)-aminocarbonyl-C₂-C₄-halogenalkenyl, 2-[(C₁-C₄-Alkoxy)carbonyl]-pyrrolidin-1-yl-carbonyl-C₂-C₄-alkenyl, 2-[(C₁-C₄-Alkoxy)carbonyl]-pyrrolidin-1-yl-carbonyl-C₂-C₄-halogenalkenyl, Formyl, (C₁-C₄-Alkyl)carbonyl, (C₁-C₄-Halogenalkyl)carbonyl, Hydroxyimino-C₁-C₄-alkyl, C₁-C₄-Alkoxyimino-C₁-C₄-alkyl, 1,1-Di-(C₁-C₄-alkoxy)-C₁-C₄-alkyl, 1,1-Di-(C₁-C₄-alkylthio)-C₁-C₄-alkyl, Nitro, Hydroxylamino, Amino, C₁-C₄-Alkylamino, C₃-C₆-Cycloalkylamino, Di-(C₁-C₄-alkyl)-amino, (C₁-C₄-Alkyl)carbonylamino, (C₁-C₄-Halogenalkyl)carbonylamino, C₁-C₄-Alkylsulfonylamino, Di-(C₁-C₄-alkylsulfonyl)-amino, C₁-C₄-Halogenalkylsulfonylamino, Hydroxy, C₁-C₄-Alkoxy, C₁-C₄-Halogenalkoxy, Cyano-C₁-C₄-alkoxy, C₁-C₄-Alkoxy-C₁-C₄-alkoxy, C₁-C₄-Alkylthio-C₁-C₄-alkoxy, C₃-C₆-Cycloalkoxy, C₂-C₄-Alkenyloxy, C₂-C₄-Alkinyloxy, Hydroxycarbonyl-C₁-C₄-alkoxy, (C₁-C₆-Alkoxy)carbonyl-C₁-C₄-alkoxy, C₁-C₄-Alkoxy-(C₁-C₄-alkoxy)carbonyl-C₁-C₄-alkoxy, (C₁-C₄-Alkoxy)carbonyl-(C₁-C₄-alkoxy)carbonyl-C₁-C₄-alkoxy, Aminocarbonyl-C₁-C₄-alkoxy, C₁-C₄-Alkylaminocarbonyl-C₁-C₄-alkoxy, Di-(C₁-C₄-alkyl)-aminocarbonyl-C₁-C₄-alkoxy, 1-Pyrrolidinyl-carbonyl-C₁-C₄-alkoxy, 1-Piperidinyl-carbonyl-C₁-C₄-alkoxy, 4-Morpholinyl-carbonyl-C₁-C₄-alkoxy, Aminocarbonyl-(C₁-C₄-alkoxy)carbonyl-C₁-C₄-alkoxy, (C₁-C₄-Alkyl)aminocarbonyl-(C₁-C₄-alkoxy)carbonyl-C₁-C₄-alkoxy, Di-(C₁-C₄-alkyl)-aminocarbonyl-(C₁-C₄-alkoxy)-carbonyl-C₁-C₄-alkoxy, 1-Pyrrolidinyl-carbonyl-(C₁-C₄-alkoxy)carbonyl-C₁-C₄-alkoxy, 1-Piperidinyl-carbonyl-(C₁-C₄-alkoxy)carbonyl-C₁-C₄-alkoxy, 4-Morpholinyl-carbonyl-(C₁-C₄-alkoxy)carbonyl-C₁-C₄-alkoxy, [(C₁-C₄-Alkoxy)carbonyl-C₁-C₄-alkyl]-aminocarbonyl-C₁-C₄-alkoxy, N-[(C₁-C₄-Alkoxy)carbonyl-C₁-C₄-alkyl]-N-(C₁-C₄-alkyl)-aminocarbonyl-C₁-C₄-alkoxy, [2-[(C₁-C₄-Alkoxy)carbonyl]-pyrrolidin-1-yl]-carbonyl-C₁-C₄-alkoxy, Cyano, Hydroxycarbonyl, COCl, (C₁-C₄-Alkoxy)carbonyl, (C₁-C₄-Halogenalkoxy)carbonyl, C₁-C₄-Alkoxy-(C₁-C₄-alkoxy)carbonyl, Aminocarbonyl, C₁-C₄-Alkylaminocarbonyl, Di-(C₁-C₄-alkyl)-aminocarbonyl, C₃-C₆-Cycloalkylaminocarbonyl, 1-Pyrrolidinylcarbonyl, 1-Piperidinylcarbonyl, 4-Morpholinylcarbonyl, Hydroxycarbonyl-C₁-C₄-alkylaminocarbonyl, N-(Hydroxycarbonyl-C₁-C₄-alkyl)-N-(C₁-C₄-alkyl)-aminocarbonyl, (C₁-C₄-Alkoxy)carbonyl-C₁-C₄-alkylaminocarbonyl, N-[(C₁-C₄-Alkoxy)carbonyl-C₁-C₄-alkyl]-N-(C₁-C₄-alkyl)-aminocarbonyl, 2-Hydroxycarbonyl-pyrrolidin-1-yl-carbonyl, 2-[(C₁-C₄-Alkoxy)carbonyl]-pyrrolidin-1-yl-carbonyl oder eine Gruppe 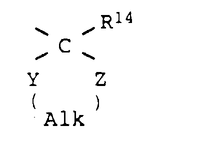 wobei
- Y und Z: unabhängig voneinander für Sauerstoff oder Schwefel,
- R¹⁴: für Wasserstoff, C₁-C₄-Alkyl oder (C₁-C₄-Alkoxy)carbonyl und
- Alk: für eine Ethylen- oder Trimethylenkette, bei denen ein oder mehrere Wasserstoffatome jeweils durch C₁-C₄-Alkyl oder (C₁-C₄-Alkoxy)carbonyl ersetzt sein können,
stehen;
- R¹²: Wasserstoff, Nitro, Amino, Hydroxylamino, C₁-C₄-Alkylsulfonylamino, Di-(C₁-C₄-alkylsulfonyl)-amino, C₁-C₄-Halogenalkylsulfonylamino, (C₁-C₄-Alkoxy)carbonyl-C₁-C₄-halogenalkyl oder (C₁-C₄-Alkoxy)carbonyl-C₂-C₄-halogenalkenyl;
mit der Maßgabe, daß R⁵, R⁷, R¹¹ und R¹³ nicht alle gleichzeitig Wasserstoff bedeuten oder R⁷ und R⁸ oder R¹⁰ und R¹¹ nicht gleichzeitig Fluor bedeuten, sowie die landwirtschaftlich brauchbaren Salze der Verbindungen I, sofern diese existieren.

Außerdem betrifft die Erfindung
- die Verwendung von Verbindungen I', wobei I' der Definition von I ohne die Maßgabe entspricht, als Herbizide und/oder zur Desiccation und/oder Defoliation von Pflanzen,
- herbizide Mittel und Mittel zur Desiccation und/oder Defoliation von Pflanzen, welche die Verbindungen I' als wirksame Substanzen enthalten,
- Verfahren zur Bekämpfung von unerwünschtem Pflanzenwuchs und zur Desiccation und/oder Defoliation von Pflanzen mit den Verbindungen I',
- Verfahren zur Herstellung der Verbindungen I und von herbiziden Mitteln und Mitteln zur Desiccation und/oder Defoliation von Pflanzen unter Verwendung der Verbindungen I', sowie
- die Verwendung von Zwischenprodukten II zur Herstellung der Verbindungen I.

2-Phenylpyridine vom Typ der Verbindungen I wurden in der Literatur bisher als Flüssigkristalle und Pharmaka beschrieben. Flüssigkristalle, deren allgemeine Formeln auch 2-Phenylpyridine umfassen, sind z.B. Gegenstand der folgenden Druckschriften: DE-A 38 07 955, DE-A 39 15 804, DE-A 42 36 103, EP-A 283 326, EP-A 433 826, EP-A 541 081, JP-A 63/048 254, JP-A 63/254 182, JP-A 63/313 768, JP-A 01/066 161, JP-A 01/070 455, JP-A 05/331 143 und Mol. Cryst. Liq. Cryst. 62(1-2), 103-114 (1980).

2-Phenylpyridine mit pharmakologischer Wirkung sind z.B. der DE-A 25 12 673, DE-A 30 35 259, BE-A 885 484, EP-A 274 654, EP-A 039 892 und WO 92/06085 zu entnehmen.

Den Verbindungen I strukturell ähnliche 2-Phenylpyridine sind außerdem in der DD-A 251 206, in J. Chem. Res., Synop. 3, 86, 1994 und Izv. Timiryazevsk. S-Kh. Akad. 3, 155-160 (1990) beschrieben worden.

Schließlich ist aus der älteren deutschen Anmeldung DE-A 43 23 916 (Veröffentlichungsdatum 19.01.95 : Stand der Technik im Sinne des Artikels 54, Absatz 3 EPÜ) bekannt, daß bestimmte 2-(4-Hydroxyphenyl)pyridine, u.a. die Verbindungen der Formel II' 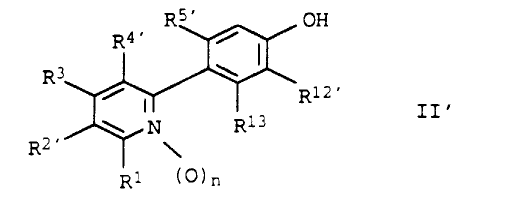 wobei
- R²': für Halogen, Cyano, Nitro, C₁-C₄-Alkyl, C₁-C₄-Halogenalkyl, C₁-C₄-Halogenalkoxy, C₁-C₄-Alkylthio oder C₁-C₄-Halogenalkylthio;
- R⁴': für Wasserstoff, Nitro, Amino, Cyano, Hydroxy, Mercapto, Hydroxycarbonyl, Halogen, C₁-C₄-Alkyl, C₁-C₄-Halogenalkyl, C₁-C₄-Alkoxy, C₁-C₄-Halogenalkoxy, C₁-C₄-Alkylamino, Di-(C₁-C₄-alkyl)-amino, C₁-C₄-Alkylthio, C₁-C₄-Halogenalkylthio oder (C₁-C₄-Alkoxy)carbonyl;
- R⁵': für Wasserstoff oder Halogen und
- R¹²': für Nitro, Amino, C₁-C₄-Alkylsulfonylamino, Di-(C₁-C₄-alkylsulfonyl)-amino, C₁-C₄-Halogenalkylsulfonylamino, (C₁-C₄-Alkoxy)carbonyl-CH(Halogen)-CH₂-, (C₁-C₄-Alkoxy)carbonyl-C(Halogen)=CH-, (C₁-C₄-Alkoxy)carbonyl-CH=C(Halogen)-oder (C₁-C₄-Alkoxy)carbonyl-C(Halogen)=C(Halogen)-
stehen, herbizid und desiccant/defoliant wirksam sind.

Die herbizide Wirkung der bekannten Verbindungen bezüglich der Schadpflanzen ist jedoch nicht immer voll befriedigend.

Aufgabe der vorliegenden Erfindung war es demnach, neue herbizid wirksame Verbindungen bereitzustellen, mit denen sich unerwünschte Pflanzen besser als bisher gezielt bekämpfen lassen. Die Aufgabe erstreckt sich auch auf die Bereitstellung neuer desiccant/defoliant wirksamer Verbindungen.

Demgemäß wurden die eingangs definierten substituierten 2-Phenylpyridine der Formel I bzw. I' sowie deren herbizide Wirkung gefunden. Ferner wurden herbizide Mittel gefunden, die die Verbindungen I' enthalten und eine sehr gute herbizide Wirkung besitzen. Außerdem wurden Verfahren zur Herstellung dieser Mittel und Verfahren zur Bekämpfung von unerwünschtem Pflanzenwuchs mit den Verbindungen I' gefunden.

Des weiteren wurde gefunden, daß die Verbindungen I' auch zur Defoliation und Desiccation von Pflanzenteilen geeignet sind, wofür Kulturpflanzen wie Baumwolle, Kartoffel, Raps, Sonnenblume, Sojabohne oder Ackerbohnen, insbesondere Baumwolle, in Betracht kommen. Diesbezüglich wurden Mittel zur Desiccation und/oder Defoliation von Pflanzen, Verfahren zur Herstellung dieser Mittel und Verfahren zur Desiccation und/oder Defoliation von Pflanzen mit den Verbindungen I gefunden.

Die Verbindungen der Formel I können je nach Substitutionsmuster ein oder mehrere Chiralitätszentren enthalten und liegen dann als Enantiomeren- oder Diastereomerengemische vor. Gegenstand der Erfindung sind sowohl die reinen Enantiomeren oder Diastereomeren als auch deren Gemische.

Die substituierten 2-Phenylpyridine I können in Form ihrer landwirtschaftlich brauchbaren Salze vorliegen, wobei es auf die Art des Salzes in der Regel nicht ankommt. Im allgemeinen kommen die Salze von solchen Basen und diejenigen Säureadditionssalze in Betracht, bei denen die herbizide Wirkung im Vergleich zu der freien Verbindung I nicht negativ beeinträchtigt ist.

Als basische Salze eignen sich besonders diejenigen der Alkalimetalle, vorzugsweise Natrium- und Kaliumsalze, der Erdalkalimetalle, vorzugsweise Calcium- und Magnesiumsalze, die der Übergangsmetalle, vorzugsweise Zink- und Eisensalze, sowie Ammoniumsalze, bei denen das Ammoniumion gewünschtenfalls ein bis drei C₁-C₄-Alkyl-, Hydroxy-C₁-C₄-alkylsubstituenten und/oder einen Phenyl- oder Benzylsubstituenten tragen kann, vorzugsweise Diisopropylammonium-, Tetramethylammonium-, Tetrabutylammonium-, Trimethylbenzylammonium- und Trimethyl- (2-hydroxyethyl)-ammoniumsalze, des weiteren Phosphoniumsalze, Sulfoniumsalze, vorzugsweise Tri-(C₁-C₄-alkyl)sulfoniumsalze, und Sulfoxoniumsalze, vorzugsweise Tri-(C₁-C₄-alkyl)sulfoxoniumsalze.

Unter den Säureadditionssalzen sind in erster Linie die Hydrochloride und -bromide, Sulfate, Nitrate, Phosphate, Oxalate und die Dodecylbenzolsulfonate zu nennen.

Die für die Substituenten R¹ bis R¹⁴ oder als Reste an diesen Substituenten oder an der Kette Alk genannten organischen Molekülteile stellen - wie die Bedeutung Halogen - Sammelbegriffe für individuelle Auf zählungen der einzelnen Gruppenmitglieder dar. Sämtliche Kohlenstoffketten, also alle Alkyl-, Halogenalkyl-, Hydroxyalkyl-, Alkoxy-, Cyanoalkoxy-, Alkylthio-, Alkylsulfinyl, Alkylsulfonyl-, Halogenalkoxy-, Halogenalkylthio-, Halogenalkylsulfonyl-, Alkylamino-, Dialkylamino-, Alkylcarbonyl-, Halogenalkylcarbonyl-, Alkoxycarbonyl-, Alkylaminocarbonyl-, Dialkylaminocarbonyl-, Aminocarbonylalkyl-, Alkenyl-, Halogenalkenyl-, Alkinyl-, Alkenyloxy-, Alkinyloxy-, Hydroxycarbonylalkyl-, Hydroxycarbonylalkoxy-, Hydroxycarbonylalkenyl-, Hydroxycarbonylalkinyl-, Hydroxyiminoalkyl-, Aminocarbonylalkoxy-, Aminocarbonylalkenyl- oder Aminocarbonylalkinyl-Teile können geradkettig oder verzweigt sein. Halogenierte Substituenten tragen vorzugsweise ein bis fünf gleiche oder verschiedene Halogenatome.

Im einzelnen stehen beispielsweise:
- Halogen für: Fluor, Chlor, Brom oder Iod;
- C₁-C₄-Alkyl sowie die Alkyl-Teile von C₁-C₄-Alkoxy-C₁-C₄-alkyl, C₁-C₄-Alkylaminocarbonyl-C₁-C₄-alkyl, C₁-C₄-Alkylaminocarbonyl-C₂-C₄-alkenyl, C₁-C₄-Alkylaminocarbonyl-C₂-C₄-alkinyl, N-(Hydroxycarbonyl-C₁-C₄-alkyl)-N-(C₁-C₄-alkyl)-aminocarbonyl-C₂-C₄-alkenyl, N-(Hydroxycarbonyl-C₁-C₄-alkyl)-N-(C₁-C₄-alkyl)-aminocarbonyl-C₂-C₄-halogenalkenyl, [(C₁-C₄-Alkoxy)carbonyl-C₁-C₄-alkyl]-aminocarbonyl-C₂-C₄-alkenyl, N-[(C₁-C₄-Alkoxy)carbonyl-C₁-C₄-alkyl]-N-(C₁-C₄-alkyl)-aminocarbonyl-C₂-C₄-alkenyl, [(C₁-C₄-Alkoxy)carbonyl-C₁-C₄-alkyl]-aminocarbonyl-C₂-C₄-halogenalkenyl, N-[(C₁-C₄-Alkoxy)carbonyl-C₁-C₄-alkyl]-N-(C₁-C₄-alkyl)-aminocarbonyl-C₂-C₄-halogenalkenyl, C₁-C₄-Alkoxyimino-C₁-C₄-alkyl, 1,1-Di-(C₁-C₄-alkoxy)-C₁-C₄-alkyl, 1,1-Di-(C₁-C₄-alkylthio)-C₁-C₄-alkyl, [(C₁-C₄-Alkoxy)carbonyl-C₁-C₄-alkyl]-aminocarbonyl-C₁-C₄-alkoxy, N-[(C₁-C₄-Alkoxy)carbonyl-C₁-C₄-alkyl]-N-(C₁-C₄-alkyl)-aminocarbonyl-C₁-C₄-alkoxy und N-(Hydroxycarbonyl-C₁-C₄-alkyl)-N-(C₁-C₄-alkyl)-aminocarbonyl, N-[(C₁-C₄-Alkoxy)carbonyl-C₁-C₄-alkyl]-N-(C₁-C₄-alkyl)-aminocarbonyl und Di-(C₁-C₄-alkyl)-aminocarbonyl-(C₁-C₄-alkoxy)carbonyl-C₁-C₄-alkoxy für:
Methyl, Ethyl, n-Propyl, 1-Methylethyl, n-Butyl, 1-Methylpropyl, 2-Methylpropyl oder 1,1-Dimethylethyl;
- C₁-C₄-Halogenalkyl sowie die Halogenalkyl-Teile von Aminocarbonyl-C₁-C₄-halogenalkyl und C₁-C₄-Alkylaminocarbonyl-C₁-C₄-halogenalkyl für: einen C₁-C₄-Alkylrest wie vorstehend genannt, der partiell oder vollständig durch Fluor, Chlor, Brom und/oder Iod substituiert ist, also z.B. Chlormethyl, Dichlormethyl, Trichlormethyl, Fluormethyl, Difluormethyl, Trifluormethyl, Chlorfluormethyl, Dichlorfluormethyl, Chlordifluormethyl, 2-Fluorethyl, 2-Chlorethyl, 2-Bromethyl, 2-Iodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Petafluorethyl, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, 1-(Fluormethyl)-2-fluorethyl, 1-(Chlormethyl) -2-chlorethyl, 1-(Brommethyl)-2-bromethyl, 4-Fluorbutyl, 4-Chlorbutyl, 4-Brombutyl oder Nonafluorbutyl;
- die Halogenalkyl-Teile von (C₁-C₄-Alkoxy)carbonyl-C₂-C₄-halogenalkyl und Di-(C₁-C₄-alkyl)-aminocarbonyl-C₂-C₄-halogenalkyl für: partiell oder vollständig durch Fluor, Chlor, Brom und/oder Iod substituiertes Ethyl, n-Propyl, 1-Methylethyl, n-Butyl, 1-Methylpropyl, 2-Methylpropyl oder 1,1-Dimethylethyl, also z.B. 2-Fluorethyl, 2-Chlorethyl, 2-Bromethyl, 2-Iodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Petafluorethyl, 2-Fluorpropyl,3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl oder Nonafluorbutyl;
- C₁-C₄-Alkoxy sowie die Alkoxy-Teile von C₁-C₄-Alkoxy-C₁-C₄-alkyl, C₁-C₄-Alkoxy-C₁-C₄-alkoxy, C₁-C₄-Alkylthio-C₁-C₄-alkoxy, (C₁-C₄-Alkoxy)carbonyl, (C₁-C₄-Alkoxy)carbonyl-C₁-C₄-alkyl, (C₁-C₄-Alkoxy)carbonyl-C₂-C₄-alkenyl, (C₁-C₄-Alkoxy)carbonyl-C₂-C₄-alkinyl, (C₁-C₄-Alkoxy)carbonyl-C₂-C₄-halogenalkyl, (C₁-C₄-Alkoxy)carbonyl-C₂-C₄-halogenalkenyl, [(C₁-C₄-Alkoxy)carbonyl-C₁-C₄-alkyl]-aminocarbonyl-C₂-C₄-alkenyl, N-[(C₁-C₄-Alkoxy)carbonyl-C₁-C₄-alkyl]-N-(C₁-C₄-alkyl)-aminocarbonyl-C₂-C₄-alkenyl, [(C₁-C₄-Alkoxy)carbonyl-C₁-C₄-alkyl]-aminocarbonyl-C₂-C₄-halogenalkenyl, N-[(C₁-C₄-Alkoxy)carbonyl-C₁-C₄-alkyl]-N-(C₁-C₄-alkyl)-aminocarbonyl-C₂-C₄-halogenalkenyl, 2-[(C₁-C₄-Alkoxy)carbonyl]-pyrrolidin-1-yl-carbonyl-C₂-C₄-alkenyl, 2-[(C₁-C₄-Alkoxy)-carbonyl]-pyrrolidin-1-yl-carbonyl-C₂-C₄-halogenalkenyl, (C₁-C₄-Alkoxy)carbonyl-C₁-C₄-alkoxy, C₁-C₄-Alkoxy-(C₁-C₄-alkoxy)carbonyl-C₁-C₄-alkoxy, (C₁-C₄-Alkoxy)carbonyl-(C₁-C₄-alkoxy)carbonyl-C₁-C₄-alkoxy, C₁-C₄-Alkoxyimino-C₁-C₄-alkyl, 1,1-Di-(C₁-C₄-alkoxy)-C₁-C₄-alkyl, C₁-C₄-Alkylaminocarbonyl-C₁-C₄-alkoxy, Di-(C₁-C₄-alkyl)-aminocarbonyl-C₁-C₄-alkoxy, [(C₁-C₄-Alkoxy)carbonyl-C₁-C₄-alkyl]-aminocarbonyl-C₁-C₄-alkoxy, N-[(C₁-C₄-Alkoxy)carbonyl-C₁-C₄-alkyl]-N-(C₁-C₄-alkyl)-aminocarbonyl-C₁-C₄-alkoxy, [2-[(C₁-C₄-Alkoxy) carbonyl]-pyrrolidin-1-yl]-carbonyl-C₁-C₄-alkoxy, C₁-C₄-Alkoxy-(C₁-C₄-alkoxy)carbonyl, (C₁-C₄-Alkoxy)carbonyl-C₁-C₄-alkylaminocarbonyl, N-[(C₁-C₄-Alkoxy)carbonyl-C₁-C₄-alkyl]-N-(C₁-C₄-alkyl)-aminocarbonyl, 2-[(C₁-C₄-Alkoxy)carbonyl]-pyrrolidin-1-yl-carbonyl, Aminocarbonyl-(C₁-C₄-alkoxy)carbonyl-C₁-C₄-alkoxy, (C₁-C₄-Alkyl)aminocarbonyl-(C₁-C₄-alkoxy)carbonyl-C₁-C₄-alkoxy, Di-(C₁-C₄-alkyl)-aminocarbonyl-(C₁-C₄-alkoxy)carbonyl-C₁-C₄-alkoxy, 1-Pyrrolidinylcarbonyl-(C₁-C₄-alkoxy)carbonyl-C₁-C₄-alkoxy, 1-Piperidinylcarbonyl-(C₁-C₄-alkoxy)carbonyl-C₁-C₄-alkoxy, 4-Morpholinylcarbonyl-(C₁-C₄-alkoxy)carbonyl-C₁-C₄-alkoxy für: Methoxy, Ethoxy, n-Propoxy, 1-Methylethoxy, n-Butoxy, 1-Methylpropoxy, 2-Methylpropoxy oder 1,1-Dimethylethoxy;
- C₁-C₄-Halogenalkoxy für: C₁-C₄-Alkoxy wie vorstehend genannt, das partiell oder vollständig durch Fluor, Chlor, Brom und/ oder Iod substituiert ist, also z.B. Difluormethoxy, Trifluormethoxy, Chlordifluormethoxy, Bromdifluormethoxy, 2-Fluorethoxy, 2-Chlorethoxy, 2-Bromethoxy, 2-Iodethoxy, 2,2-Difluorethoxy, 2,2,2-Trifluorethoxy, 2-Chlor-2-fluorethoxy, 2-Chlor-2,2-difluorethoxy, 2,2-Dichlor-2-fluorethoxy, 2,2,2-Trichlorethoxy, Pentafluorethoxy, 2-Fluorpropoxy, 3-Fluorpropoxy, 2-Chlorporpoxy, 3-Chlorpropoxy, 2-Brompropoxy, 3-Brompropoxy, 2,2-Difluorpropoxy, 2,3-Difluorpropoxy, 2,3-Dichlorpropoxy, 3,3,3-Trifluorpropoxy, 3,3,3-Trichlorpropoxy, 2,2,3,3,3-Pentafluorpropoxy, Heptafluorporpoxy, 1-(Fluormethyl)-2-fluorethoxy, 1-(Chlormethyl)-2-chlorethoxy, 1-(Brommethyl)-2-bromethoxy, 4-Fluorbutoxy, 4-Chlorbutoxy, 4-Brombutoxy oder Nonafluorbutoxy;
- Cyano-C₁-C₄-alkoxy für: Cyanomethoxy, 1-Cyanoeth-1-yloxy, 2-Cyanoeth-1-yloxy, 1-Cyanoprop-1-yloxy, 2-Cyanoprop-1-yloxy, 3-Cyanoprop-1-yloxy, 1-Cyanoprop-2-yloxy, 2-Cyanoprop-2-yloxy, 1-Cyanobut-1-yloxy, 2-Cyanobut-1-yloxy, 3-Cyanobut-1-yloxy, 4-Cyanobut-1-yloxy, 1-Cyanobut-2-yloxy, 2-Cyano-but-2-yloxy, 1-Cyanobut-3-yloxy, 2-Cyanobut-3-yloxy, 1-Cyano-2-methyl-prop-3-yloxy, 2-Cyano-2-methyl-prop-3-yloxy, 3-Cyano-2-methyl-prop-3-yloxy oder 2-Cyano-methyl-prop-2-yloxy;
- C₁-C₄-Alkylthio sowie die Alkylthio-Teile von 1,1-Di-(C₁-C₄-alkylthio)-C₁-C₄-alkyl und C₁-C₄-Alkylthio-C₁-C₄-alkoxy für: Methylthio, Ethylthio, n-Propylthio, 1-Methylethylthio, n-Butylthio, 1-Methylpropylthio, 2-Methylpropylthio oder 1,1-Dimethylethylthio;
- C₁-C₄-Halogenalkylthio für: C₁-C₄-Alkylthio wie vorstehend genannt, das partiell oder vollständig durch Fluor, Chlor, Brom und/oder Iod substituiert ist, also z.B. Difluormethylthio, Trifluormethylthio, Chlordifluormethylthio, Bromdifluormethylthio, 2-Fluorethylthio, 2-Chlorethylthio, 2-Bromethylthio, 2-Iodethylthio, 2,2-Difluorethylthio, 2,2,2-Trifluorethylthio, 2,2,2-Trichlorethylthio, 2-Chlor-2-fluorethylthio, 2-Chlor-2,2-difluorethylthio, 2,2,-Dichlor-2-fluorethylthio, Pentafluorethylthio, 2-Fluorpropylthio, 3-Fluorpropylthio, 2-Chlorpropylthio, 3-Chlorpropylthio, 2-Brompropylthio, 3-Brompropylthio, 2,2,-Difluorpropylthio, 2,3-Difluorpropylthio, 2,3-Dichlorpropylthio, 3,3,3-Trifluorpropylthio, 3,3,3-Trichlorpropylthio, 2,2,3,3,3-Pentafluorpropylthio, Heptafluorpropylthio, 1-(Fluormethyl)-2-fluorethylthio, 1-(Chlormethyl)-2-chlorethylthio, 1-(Brommethyl)-2-bromethylthio, 4-Fluorbutylthio, 4-Chlorbutylthio, 4-Brombutylthio oder Nonafluorbutylthio;
- C₁-C₄-Alkylamino sowie die Alkylamino-Teile von C₁-C₄-Alkylaminocarbonyl, C₁-C₄-Alkylaminocarbonyl-C₁-C₄-halogenalkyl, C₁-C₄-Alkylaminocarbonyl-C₂-C₄-halogenalkenyl, C₁-C₄-Alkylaminocarbonyl-C₁-C₄-alkoxy, (C₁-C₄-Alkoxy)carbonyl-C₁-C₄-alkylaminocarbonyl und (C₁-C₄-Alkyl) aminocarbonyl-(C₁-C₄-alkoxy)carbonyl-C₁-C₄-alkoxy für: Methylamino, Ethylamino, n-Propylamino, 1-Methylethylamino, n-Butylamino, 1-Methyl-propylamino, 2-Methylpropylamino oder 1,1-Dimethylethylamino;
- Di-(C₁-C₄-alkyl)-amino sowie die Dialkylamino-Teile von Di-(C₁-C₄-alkyl)-aminocarbonyl, Di-(C₁-C₄-alkyl)-aminocarbonyl-C₁-C₄-alkyl, Di-(C₁-C₄-alkyl)-aminocarbonyl-C₂-C₄-alkenyl, Di-(C₁-C₄-alkyl)-aminocarbonyl-C₂-C₄-alkinyl, Di-(C₁-C₄-alkyl)-aminocarbonyl-C₂-C₄-halogenalkyl, Di-(C₁-C₄-alkyl)-aminocarbonyl-C₂-C₄-halogenalkenyl und Di-(C₁-C₄-alkyl)-aminocarbonyl-C₁-C₄-alkoxy für: z. B. N,N-Dimethylamino, N,N-Diethylamino, N,N-Diisopropylamino, N,N-Dibutylamino, N,N-Di-(1-methylpropyl)amino, N,N-Di-(2-methylpropyl)amino, N,N-Di-(1,1-dimethylethyl)amino, N-Ethyl-N-methylamino, N-Methyl-N-propylamino, N-Methyl-N-(1-methylethyl)amino, N-Butyl-N-methylamino, N-Methyl-N-(1-methylpropyl) amino-, N-Methyl-N-(2-methylproyl)amino, N-(1,1-Dimethylethyl)-N-methylamino, N-Ethyl-N-propylamino, N-Ethyl-N-(1-methylethyl)amino, N-Butyl-N-ethylamino, N-Ethyl-N-(1-methylpropyl)amino, N-Ethyl-N-(2-methylpropyl)amino, N-Ethyl-N-(1,1-dimethylethyl)amino, N-(1-Methyletyl)-N-propylamino, N-Butyl-N-propylamino, N-(1-Methylpropyl)-N-propylamino, N-(2-Methylpropyl)-N-propylamino, N-(1,1-Dimethylethyl)-N-propylamino, N-Butyl-N-(1-methylethyl)amino, N-(1-Methylethyl)-N-(1-methylpropyl)amino, N-(1-Methylethyl)-N-(2-methylpropyl)amino, N-(1,1,-Dimethylethyl)-N-(1-methylethyl)amino, N-Butyl-N-(1-methylpropyl)amino-, N-Butyl-N-(2-methylpropyl)amino, N-Butyl-N-(1,1-dimethylethyl)amino, N-(1-Methylpro-pyl)-N-(2-methylpropyl)amino, N-(1,1-Dimethylethyl)-N-(1-methylpropyl)amino oder N-(1,1-Dimethylethyl)-N-(2-methylpropyl)amino;
- C₁-C₄-Alkylsulfinyl für: Methylsulfinyl, Ethylsulfinyl, n-Propylsulfinyl, 1-Methylethylsulfinyl, n-Butylsulfinyl, 1-Methyl-propylsulfinyl, 2-Methylpropylsulfinyl oder 1,1-Dimethylethylsulfinyl;
- C₁-C₄-Halogenalkylsulfinyl für: C₁-C₄-Alkylsulfinyl wie vorstehend genannt, das partiell oder vollständig durch Fluor, Chlor, Brom und/oder Iod substituiert ist, also z.B. Difluormethylsulfinyl, Trifluormethylsulfinyl, Chlordifluormethylsulfinyl, Bromdifluormethylsulfinyl, 2-Fluorethylsulfinyl, 2-Chlorethylsulfinyl, 2-Bromethylsulfinyl, 2-Iodethylsulfinyl, 2,2-Difluorethylsulfinyl, 2,2,2-Trifluorethylsulfinyl, 2,2,2-Trichlorethylsulfinyl, 2-Chlor-2-fluorethylsulfinyl, 2-Chlor-2,2-difluorethylsulfinyl, 2,2,-Dichlor-2-fluorethylsulfinyl, Pentafluorethylsulfinyl, 2-Fluorpropylsulfinyl, 3-Fluorpropylsulfinyl, 2-Chlorpropylsulfinyl, 3-Chlorpropylsulfinyl, 2-Brompropylsulfinyl, 3-Brompropylsulfinyl, 2,2,-Difluorpropylsulfinyl, 2,3-Difluorpropylsulfinyl, 2,3-Dichlorpropylsulfinyl, 3,3,3-Trifluorpropylsulfinyl, 3,3,3-Trichlorpropylsulfinyl, 2,2,3,3,3-Pentafluorpropylsulfinyl, Heptafluorpropylsulfinyl, 1-(Fluormethyl)-2-fluorethylsulfinyl, 1-(Chlormethyl)-2-chlorethylsulfinyl, 1-(Brommethyl)-2-bromethylsulfinyl, 4-Fluorbutylsulfinyl, 4-Chlorbutylsulfinyl, 4-Brombutylsulfinyl oder Nonafluorbutylsulfinyl;
- C₁-C₄-Alkylsulfonyl sowie die Alkylsulfonyl-Teile von C₁-C₄-Alkylsulfonylamino und Di-(C₁-C₄-alkylsulfonyl)-amino für: Methylsulfonyl, Ethylsulfonyl, n-Propylsulfonyl, 1-Methylethylsulfonyl, n-Butylsulfonyl, 1-Methylpropylsulfonyl, 2-Methylpropylsulfonyl oder 1,1-Dimethylethylsulfonyl;
- C₁-C₄-Halogenalkylsulfonyl sowie der Halogenalkylsulfonyl-Teil von C₁-C₄-Halogenalkylsulfonylamino für: C₁-C₄-Alkylsulfonyl wie vorstehend genannt, das partiell oder vollständig durch Fluor, Chlor, Brom und/oder Iod substituiert ist, also z.B. Difluormethylsulfonyl, Trifluormethylsulfonyl, Chlordifluormethylsulfonyl, Bromdifluormethylsulfonyl, 2-Fluorethylsulfonyl, 2-Chlorethylsulfonyl, 2-Bromethylsulfonyl, 2-Iodethylsulfonyl, 2,2-Difluorethylsulfonyl, 2,2,2-Trifluorethylsulfonyl, 2,2,2-Trichlorethylsulfonyl, 2-Chlor-2-fluorethylsulfonyl, 2-Chlor-2,2-difluorethylsulfonyl, 2,2,-Dichlor-2-fluorethylsulfonyl, Pentafluorethylsulfonyl, 2-Fluorpropylsulfonyl, 3-Fluorpropylsulfonyl, 2-Chlorpropylsulfonyl, 3-Chlorpropylsulfonyl, 2-Brompropylsulfonyl, 3-Brompropylsulfonyl, 2,2,-Difluorpropylsulfonyl, 2,3-Difluorpropylsulfonyl, 2,3-Dichlorpropylsulfonyl, 3,3,3-Trifluorpropylsulfonyl, 3,3,3-Trichlorpropylsulfonyl, 2,2,3,3,3-Pentafluorpropylsulfonyl, Heptafluorpropylsulfonyl, 1-(Fluormethyl)-2-fluorethylsulfonyl, 1-(Chlormethyl)-2-chlorethylsulfonyl, 1-(Brommethyl)-2-bromethylsulfonyl, 4-Fluorbutylsulfonyl, 4-Chlorbutylsulfonyl, 4-Brombutylsulfonyl oder Nonafluorbutylsulfonyl;
- C₁-C₄-Hydroxyalkyl für: Hydroxymethyl, 1-Hydroxyeth-1-yl, 2-Hydroxyeth-1-yl, 1-Hydroxyprop-1-yl, 2-Hydroxyprop-1-yl, 3-Hydroxyprop-1-yl, 1-Hydroxyprop-2-yl, 2-Hydroxyprop-2-yl, 1-Hydroxybut-1-yl, 2-Hydroxybut-1-yl, 3-Hydroxybut-1-yl, 4-Hydroxybut-1-yl, 1-Hydroxybut-2-yl, 2-Hydroxy-but-2-yl, 1-Hydroxybut-3-yl, 2-Hydroxybut-3-yl, 1-Hydroxy-2-methylprop-3-yl, 2-Hydroxy-2-methyl-prop-3-yl, 3-Hydroxy-2-methylprop-3-yl oder 2-Hydroxy-methyl-prop-2-yl;
- Hydroxyimino-C₁-C₄-alkyl für: Hydroxyiminomethyl, 1-(Hydroxyimino)eth-1-yl, 2-(Hydroxyimino)eth-1-yl, 1-(Hydroxyimino)prop-1-yl, 2-(Hydroxyimino)prop-1-yl, 3-(Hydroxyimino)prop-1-yl, 1-(Hydroxyimino)prop-2-yl, 2-(Hydroxyimino)prop-2-yl, 1-(Hydroxyimino)but-1-yl, 2-(Hydroxyimino)but-1-yl, 3-(Hydroxyimino)but-1-yl, 4-(Hydroxyimino)but-1-yl, 1-(Hydroxyimino)but-2-yl, 2-(Hydroxyimino)-but-2-yl, 1-(Hydroxyimino)but-3-yl, 2-(Hydroxyimino)but-3-yl, 1-(Hydroxyimino)-2-methyl-prop-3-yl, 2-(Hydroxyimino)-2-methyl-prop-3-yl, 3-(Hydroxyimino)-2-methyl-prop-3-yl oder 2-(Hydroxyimino)-methyl-prop-2-yl;
- die Hydroxycarbonyl-C₁-C₄-alkyl-Teile von Hydroxycarbonyl-C₁-C₄-alkylaminocarbonyl und N-(Hydroxycarbonyl-C₁-C₄-alkyl)-N-(C₁-C₄-alkyl)-aminocarbonyl für: Hydroxycarbonylmethyl, 1-(Hydroxycarbonyl)eth-1-yl, 2-(Hydroxycarbonyl)eth-1-yl, 1-(Hydroxycarbonyl)prop-1-yl, 2-(Hydroxycarbonyl)prop-1-yl, 3-(Hydroxycarbonyl)prop-1-yl, 1-(Hydroxycarbonyl)prop-2-yl, 2-(Hydroxycarbonyl)prop-2-yl, 1-(Hydroxycarbonyl)but-1-yl, 2-(Hydroxycarbonyl)but-1-yl, 3-(Hydroxycarbonyl)but-1-yl, 4-(Hydroxycarbonyl)but-1-yl, 1-(Hydroxycarbonyl)but-2-yl, 2-(Hydroxycarbonyl)-but-2-yl, 1-(Hydroxycarbonyl)but-3-yl, 2-(Hydroxycarbonyl)but-3-yl, 1-(Hydroxycarbonyl)-2-methyl-prop-3-yl, 2-(Hydroxycarbonyl)-2-methyl-prop-3-yl, 3-(Hydroxycarbonyl)-2-methyl-prop-3-yl oder 2-(Hydroxycarbonyl)-methyl-prop-2-yl;
- C₃-C₆-Cycloalkyl für: Cyclopropyl, Cyclobutyl, Cyclopentyl oder Cyclohexyl;
- C₃-C₆-Cycloalkoxy für: Cyclopropyloxy, Cyclobutyloxy, Cyclopentyloxy oder Cyclohexyloxy;
- C₃-C₆-Cycloalkylamino für: Cyclopropylamino, Cyclobutylamino, Cyclopentylamino oder Cyclohexylamino;
- C₃-C₆-Cycloalkylaminocarbonyl für: Cyclopropylaminocarbonyl, Cyclobutylaminocarbonyl, Cyclopentylaminocarbonyl oder Cyclohexylaminocarbonyl;
- C₂-C₄-Alkenyl sowie die Alkenyl-Teile von C₁-C₄-Alkylaminocarbonyl-C₂-C₄-alkenyl, Di-(C₁-C₄-alkyl)-aminocarbonyl-C₂-C₄-alkenyl, (Hydroxycarbonyl-C₁-C₄-alkyl)-aminocarbonyl-C₂-C₄-alkenyl, N-(Hydroxycarbonyl-C₁-C₄-alkyl)-N-(C₁-C₄-alkyl)-aminocarbonyl-C₂-C₄-alkenyl, [(C₁-C₄-Alkoxy)carbonyl-C₁-C₄-alkyl]-aminocarbonyl-C₂-C₄-alkenyl, N-[(C₁-C₄-Alkoxy)carbonyl-C₁-C₄-alkyl]-N-(C₁-C₄-alkyl)-aminocarbonyl-C₂-C₄-alkenyl und 2-[(C₁-C₄-Alkoxy)carbonyl]-pyrrolidin-1-yl-carbonyl-C₂-C₄-alkenyl für: Vinyl, Prop-1-en-1-yl, Prop-2-en-1-yl, 1-Methylethenyl, n-Buten-1-yl, n-Buten-2-yl, n-Buten-3-yl, l-Methyl-prop-1-en-1-yl, 2-Methyl-prop-1-en-1-yl, 1-Methyl-prop-2-en-1-yl oder 2-Methyl-prop-2-en-1-yl;
- die Halogenalkenylteile von (C₁-C₄-Alkoxy)carbonyl-C₂-C₄-halogenalkenyl, Aminocarbonyl-C₂-C₄-halogenalkenyl, C₁-C₄-Alkylaminocarbonyl-C₂-C₄-halogenalkenyl, Di-(C₁-C₄-alkyl)-aminocarbonyl-C₂-C₄-halogenalkenyl, (Hydroxycarbonyl-C₁-C₄-alkyl)-aminocarbonyl-C₂-C₄-halogenalkenyl, N- (Hydroxycarbonyl-C₁-C₄-alkyl)-N-(C₁-C₄-alkyl)-aminocarbonyl-C₂-C₄-halogenalkenyl, [(C₁-C₄-Alkoxy)carbonyl-C₁-C₄-alkyl]-aminocarbonyl-C₂-C₄-halogenalkenyl, N-[(C₁-C₄-Alkoxy)carbonyl-C₁-C₄-alkyl]-N-(C₁-C₄-alkyl)-aminocarbonyl-C₂-C₄-halogenalkenyl und 2-[(C₁-C₄-Alkoxy)carbonyl]-pyrrolidin-1-yl-carbonyl-C₂-C₄-halogenalkenyl für: einen C₂-C₄-Alkenylrest wie vorstehend genannt, der partiell oder vollständig durch Fluor, Chlor, Brom und/oder Iod substituiert ist, also z.B. 2-Chlorallyl, 3-Chlorallyl oder 3,3-Dichlorallyl;
- C₂-C₄-Alkinyl sowie die Alkinyl-Teile von C₁-C₄-Alkylaminocarbonyl-C₂-C₄-alkinyl und Di-(C₁-C₄-alkyl)-aminocarbonyl-C₂-C₄-alkinyl für: Ethinyl, Prop-1-in-1-yl, Prop-2-in-1-yl, But-1-in-1-yl, But-1-in-3-yl, But-1-in-4-yl oder But-2-in-1-yl;
- C₂-C₄-Alkenyloxy für: Vinyloxy, Prop-1-en-1-yloxy, Prop-2-en-1-yloxy, 1-Methylethenyloxy, n-Buten-1-yloxy, n-Buten-2-yloxy, n-Buten-3-yloxy, 1-Methyl-prop-1-en-1-yloxy, 2-Methyl-prop-1-en-1-yloxy, 1-Methyl-prop-2-en-1-yloxy oder 2-Methyl-prop-2-en-1-yloxy;
- C₂-C₄-Alkinyloxy für: Ethinyloxy, Prop-1-in-1-yloxy, Prop-2-in-1-yloxy, But-1-in-1-yloxy, But-1-in-3-yloxy, But-1-in-4-yloxy oder But-2-in-1-yloxy;
- Hydroxycarbonyl-C₁-C₄-alkyl sowie die Hydroxycarbonylalkyl-Teile von (Hydroxycarbonyl-C₁-C₄-alkyl)-aminocarbonyl-C₂-C₄-alkenyl, (Hydroxycarbonyl-C₁-C₄-alkyl)-aminocarbonyl-C₂-C₄-halogenalkenyl, N-(Hydroxycarbonyl-C₁-C₄-alkyl)-N-(C₁-C₄-alkyl)-aminocarbonyl-C₂-C₄-alkenyl und N-(Hydroxycarbonyl-C₁-C₄-alkyl)-N- (C₁-C₄-alkyl)-aminocarbonyl-C₂-C₄-halogenalkenyl für: Hydroxycarbonylmethyl, 1-(Hydroxycarbonyl)eth-1-yl, 2-(Hydroxycarbonyl)eth-1-yl, 1-(Hydroxycarbonyl)prop-1-yl, 2-(Hydroxycarbonyl)prop-1-yl, 3-(Hydroxycarbonyl)prop-1-yl, 1-(Hydroxycarbonyl)prop-2-yl, 2-(Hydroxycarbonyl)prop-2-yl, 1-(Hydroxycarbonyl)but-1-yl, 2-(Hydroxycarbonyl)but-1-yl, 3-(Hydroxycarbonyl)but-1-yl, 4-(Hydroxycarbonyl)but-1-yl, 1-(Hydroxycarbonyl)but-2-yl, 2-(Hydroxycarbonyl)but-2-yl, 1-(Hydroxycarbonyl)but-3-yl, 2-(Hydroxycarbonyl)but-3-yl, 1-(Hydroxycarbonyl)-2-methyl-prop-3-yl, 2-(Hydroxycarbonyl)-2-methyl-prop-3-yl, 3- (Hydroxycarbonyl)-2-methyl-prop-3-yl, 2-(Hydroxycarbonylmethyl)-prop-2-yl;
- Hydroxycarbonyl-C₂-C₄-alkenyl für: z.B. 2-(Hydroxycarbonyl)vinyl, 2-(Hydroxycarbonyl)allyl, 3-(Hydroxycarbonyl)allyl oder 4-(Hydroxycarbonyl)but-2-yl;
- Hydroxycarbonyl-C₂-C₄-alkinyl für: z.B. 2-Hydroxycarbonyl)ethinyl, 3-Hydroxycarbonyl-1-propinyl oder 3-Hydroxycarbonyl-2-propin-1-yl;
- Aminocarbonyl-C₁-C₄-alkyl für: Aminocarbonylmethyl, 1- (Aminocarbonyl)eth-1-yl, 2-(Aminocarbonyl)eth-1-yl, 1-(Aminocarbonyl)prop-1-yl, 2-(Aminocarbonyl)prop-1-yl, 3-(Aminocarbonyl)prop-1-yl, 1-(Aminocarbonyl)prop-2-yl, 2-(Aminocarbonyl)prop-2-yl, 1-(Aminocarbonyl)but-1-yl, 2-(Aminocarbonyl)but-1-yl, 3-(Aminocarbonyl)but-1-yl, 4-(Aminocarbonyl)but-1-yl, 1-(Aminocarbonyl)but-2-yl, 2-(Aminocarbonyl)but-2-yl, 1-(Aminocarbonyl)but-3-yl, 2-(Aminocarbonyl)but-3-yl, 1-(Aminocarbonyl)-2-methyl-prop-3-yl, 2-(Aminocarbonyl)-2-methyl-prop-3-yl, 3-(Aminocarbonyl)-2-methyl-prop-3-yl oder 2-(Aminocarbonylmethyl)-prop-2-yl;
- Aminocarbonyl-C₁-C₄-alkoxy für: Aminocarbonylmethoxy, 1-(Aminocarbonyl)eth-1-yloxy, 2-(Aminocarbonyl)eth-1-yloxy, 1-(Aminocarbonyl)prop-1-yloxy, 2-(Aminocarbonyl)prop-1-yloxy, 3-(Aminocarbonyl)prop-1-yloxy, 1-(Aminocarbonyl)prop-2-yloxy, 2-(Aminocarbonyl)prop-2-yloxy, 1-(Aminocarbonyl)but-1-yloxy, 2-(Aminocarbonyl)but-1-yloxy, 3-(Aminocarbonyl)but-1-yloxy, 4-(Aminocarbonyl)but-1-yloxy, 1-(Aminocarbonyl)but-2-yloxy, 2-(Aminocarbonyl)but-2-yloxy, 1-(Aminocarbonyl)but-3-yloxy, 2-(Aminocarbonyl)but-3-yloxy, 1-(Aminocarbonyl)-2-methyl-prop-3-yloxy, 2-(Aminocarbonyl)-2-methyl-prop-3-yloxy, 3-(Aminocarbonyl)-2-methyl-prop-3-yloxy oder 2-(Aminocarbonylmethyl)-prop-2-yloxy;
- Aminocarbonyl-C₂-C₄-alkenyl für: z.B. 2-(Aminocarbonyl)vinyl, 2-(Aminocarbonyl)allyl, 3-(Aminocarbonyl)allyl oder 4-(Aminocarbonyl)but-2-yl;
- Aminocarbonyl-C₂-C₄-alkinyl für: z.B. 2-(Aminocarbonyl)ethinyl, 3-Aminocarbonyl-1-propinyl oder 3-Aminocarbonyl-2-propin-1-yl;
- (C₁-C₄-Alkyl)carbonyl sowie der Alkylcarbonyl-Teil von (C₁-C₆-Alkyl)carbonylamino für: Methylcarbonyl, Ethylcarbonyl, n-Propylcarbonyl, 1-Methylethylcarbonyl, n-Butylcarbonyl, 1-Methyl-propylcarbonyl, 2-Methylpropylcarbonyl oder 1,1-Dimethylethylcarbonyl;
- (C₁-C₄-Halogenalkyl)carbonyl sowie der Alkylcarbonyl-Teil von (C₁-C₆-Alkyl)carbonylamino für: (C₁-C₄-Alkyl)carbonyl wie voranstehend genannt, das partiell oder vollsltändig durch Fluor, Chlor und/oder Brom substituiert ist, also z.B. Chloracetyl, Dichloracetyl, Trichloracetyl, Fluoracetyl, Difluoracetyl, Trifluoracetyl, Chlorfluoracetyl, Dichlorfluoractyl, Chlordifluoracetyl, 1-Fluorpropionyl, 2-Fluorpropionyl, 2,2-Difluorpropionyl, 3,3,3-Trifluorpropionyl, 3-Chlor-3-fluorpropionyl, 3-Chlor-3,3-difluorpropionyl, 3,3-Dichlor-3-fluorpropionyl, Tri-chlorpropionyl oder Pentafluorpropionyl;
- Hydroxycarbonyl-C₁-C₄-alkoxy für: Hydroxycarbonylmethoxy, 1- (Hydroxycarbonyl)eth-1-yloxy, 2- (Hydroxycarbonyl)eth-1-yloxy, 1-(Hydroxycarbonyl)prop-1-yloxy, 2-(Hydroxycarbonyl)prop-1-yloxy, 3-(Hydroxycarbonyl)prop-1-yloxy, 1-(Hydroxycarbonyl)prop-2-yloxy, 2-(Hydroxcarbonyl)prop-2-yloxy, 1-(Hydroxycarbonyl)but-1-yloxy, 2-(Hydroxycarbonyl)-but-1-yloxy, 3-(Hydroxycarbonyl)but-1-yloxy, 4-(Hydroxycarbonyl)but-1-yloxy, 1-(Hydroxycarbonyl)but-2-yloxy, 2-(Hydroxycarbonyl)but-2-yloxy, 1-(Hydroxycarbonyl)but-3-yloxy, 2-(Hydroxycarbonyl)but-3-yloxy, 1-(Hydroxycarbonyl)-2-methyl-prop-3-yloxy, 2-(Hydroxycarbonyl)-2-methyl-prop-3-yloxy, 3-(Hydroxycarbonyl)-2-methyl-prop-3-yloxy oder 2-(Hydroxycarbonylmethyl)-prop-2-yloxy;
- der Aminocarbonyl-(C₁-C₄-alkoxy)-Teil von Aminocarbonyl-(C₁-C₄-alkoxy)carbonyl-C₁-C₄-alkoxy für: Aminocarbonylmethoxy, 1- (Aminocarbonyl)eth-1-yloxy, 2-(Aminocarbonyl)eth-1-yloxy, 1-(Aminocarbonyl)prop-1-yloxy, 2-(Aminocarbonyl)prop-1-yloxy, 3-(Aminocarbonyl)prop-1-yloxy, 1-(Aminocarbonyl)prop-2-yloxy, 2-(Aminocarbonyl)prop-2-yloxy, 1-(Aminocarbonyl)but-1-yloxy, 2-(Aminocarbonyl)but-1-yloxy, 3-(Aminocarbonyl)but-1-yloxy, 4-(Aminocarbonyl)but-1-yloxy, 1-(Aminocarbonyl)but-2-yloxy, 2-(Aminocarbonyl)but-2-yloxy, 1-(Aminocarbonyl)but-3-yloxy, 2-(Aminocarbonyl)but-3-yloxy, 1-(Aminocarbonyl)-2-methyl-prop-3-yloxy, 2-(Aminocarbonyl)-2-methyl-prop-3-yloxy, 3-(Aminocarbonyl)-2-methyl-prop-3-yloxy oder 2-(Aminocarbonylmethyl)-prop-2-yloxy ;
- 1-Pyrrolidinyl-carbonyl-C₁-C₄-alkoxy sowie der 1-Pyrrolidinyl-carbonylalkoxy-Teil von 1-Pyrrolidinyl-carbonyl-(C₁-C₄-alkoxy)carbonyl-C₁-C₄-alkoxy für: 1-Pyrrolidinyl-carbonylmethoxy, 1-(1-Pyrrolidinyl-carbonyl)eth-1-yloxy, 2-(1-Pyrrolidinyl-carbonyl)eth-1-yloxy, 1-(1-Pyrrolidinyl-carbonyl)prop-1-yloxy, 2-(1-Pyrrolidinyl-carbonyl)prop-1-yloxy, 3-(1-Pyrrolidinyl-Carbonyl)prop-1-yloxy, 1-(1-Pyrrolidinyl-carbonyl)prop-2-yloxy, 2-(1-Pyrrolidinyl-carbonyl)prop-2-yloxy, 1-(1-Pyrrolidinyl-carbonyl)but-1-yloxy, 2-(1-Pyrrolidinyl-carbonyl)but-1-yloxy, 3-(1-Pyrrolidinyl-carbonyl)but-1-yloxy, 4-(1-Pyrrolidinyl-carbonyl)but-1-yloxy, 1-(1-Pyrrolidinyl-carbonyl)but-2-yloxy, 2-(1-Pyrrolidinyl-carbonyl)but-2-yloxy, 1-(1-Pyrrolidinyl-carbonyl)but-3-yloxy, 2-(1-Pyrrolidinyl-carbonyl)but-3-yloxy, 1-(1-Pyrrolidinyl-carbonyl)-2-methyl-prop-3-yloxy, 2-(1-Pyrrolidinyl-carbonyl)-2-methyl-prop-3-yloxy, 3-(1-Pyrrolidinyl-carbonyl)-2-methyl-prop-3-yloxy oder 2-(1-Pyrrolidinyl-carbonylmethyl)-prop-2-yloxy;
- 1-Piperidinyl-carbonyl-C₁-C₄-alkoxy sowie der 1-Piperidinyl-carbonylalkoxy-Teil von 1-Piperidinyl-carbonyl-(C₁-C₄-alkoxy)carbonyl-C₁-C₄-alkoxy für: 1-Piperidinyl-carbonylmethoxy, 1-(1-Piperidinyl-carbonyl)eth-1-yloxy, 2-(1-Piperidinyl-carbonyl)eth-1-yloxy, 1-(1-Piperidinyl-carbonyl)prop-1-yloxy, 2-(1-Piperidinyl-carbonyl)prop-1-yloxy, 3-(1-Piperidinyl-carbonyl)prop-1-yloxy, 1-(1-Piperidinyl-carbonyl)prop-2-yloxy, 2-(1-Piperidinyl-carbonyl)prop-2-yloxy, 1-(1-Piperidinyl-carbonyl)but-1-yloxy, 2-(1-Piperidinyl-carbonyl)but-1-yloxy, 3-(1-Piperidinyl-carbonyl)but-1-yloxy, 4-(1-Piperidinyl-carbonyl)but-1-yloxy, 1-(1-Piperidinyl-carbonyl)but-2-yloxy, 2-(1-Piperidinyl-carbonyl)but-2-yloxy, 1-(1-Piperidinyl-carbonyl)but-3-yloxy, 2-(1-Piperidinyl-carbonyl)but-3-yloxy, 1-(1-Piperidinyl-carbonyl)-2-methyl-prop-3-yloxy, 2-(1-Piperidinyl-carbonyl)-2-methyl-prop-3-yloxy, 3-(1-Piperidinyl-carbonyl)-2-methyl-prop-3-yloxy oder 2-(1-Piperidinyl-carbonylmethyl)-prop-2-yloxy;
- 4-Morpholinyl-carbonyl-C₁-C₄-alkoxy sowie der 4-Morpholinyl-carbonylalkoxy-Teil von 4-Morpholinyl-carbonyl-(C₁-C₄-alkoxy)carbonyl-C₁-C₄-alkoxy für: 4-Morpholinyl-carbonylmethoxy, 1-(4-Morpholinyl-carbonyl)eth-1-yloxy, 2-(4-Morpholinyl-carbonyl)eth-1-yloxy, 1-(4-Morpholinyl-carbonyl)prop-1-yloxy, 2-(4-Morpholinyl-carbonyl)prop-1-yloxy, 3-(4-Morpholinyl-carbonyl)prop-1-yloxy, 1-(4-Morpholinyl-carbonyl)prop-2-yloxy, 2-(4-Morpholinyl-carbonyl)prop-2-yloxy, 1-(4-Morpholinyl-carbonyl)but-1-yloxy, 2-(4-Morpholinyl-carbonyl)but-1-yloxy, 3-(4-Morpholinyl-carbonyl)but-1-yloxy, 4-(4-Morpholinyl-carbonyl)but-1-yloxy, 1-(4-Morpholinyl-carbonyl)but-2-yloxy, 2-(4-Morpholinyl-carbonyl)but-2-yloxy, 1-(4-Morpholinyl-carbonyl)but-3-yloxy, 2-(4-Morpholinyl-carbonyl)but-3-yloxy, 1-(4-Morpholinyl-carbonyl)-2-methyl-prop-3-yloxy, 2-(4-Morpholinyl-carbonyl)-2-methyl-prop-3-yloxy, 3-(4-Morpholinyl-carbonyl)-2-methyl-prop-3-yloxy oder 2-(4-Morpholinyl-carbonylmethyl)-prop-2-yloxy.

Im Hinblick auf die Verwendung der erfindungsgemäßen Verbindungen der Formel I bzw. I' als Herbizide und/oder als defoliant/desiccant wirksame Verbindungen haben die Variablen vorzugsweise folgende Bedeutungen, und zwar jeweils für sich allein oder in Kombination:
- R¹: Wasserstoff;
- R²: C₁-C₄-Halogenalkyl, insbesondere Trifluormethyl;
- R³: Wasserstoff;
- R⁴: Halogen, insbesondere Chlor;
- R⁵: Wasserstoff, Fluor oder Chlor;
- R⁶: Wasserstoff;
- R⁷, R⁸, R⁹, R¹⁰: Wasserstoff, Fluor, Chlor oder Methyl;
- R¹¹: alle Bedeutungen mit Ausnahme von Wasserstoff, Cyano und Halogen;
- R¹³: Wasserstoff.

Besonders bevorzugt sind die in der folgenden Tabelle 1 aufgeführten Verbindungen Ia (≙ I mit n = Null; R¹, R³, R⁵ bis R¹⁰, R¹² und R¹³ = Wasserstoff; R² = Trifluormethyl; R⁴ = Chlor):

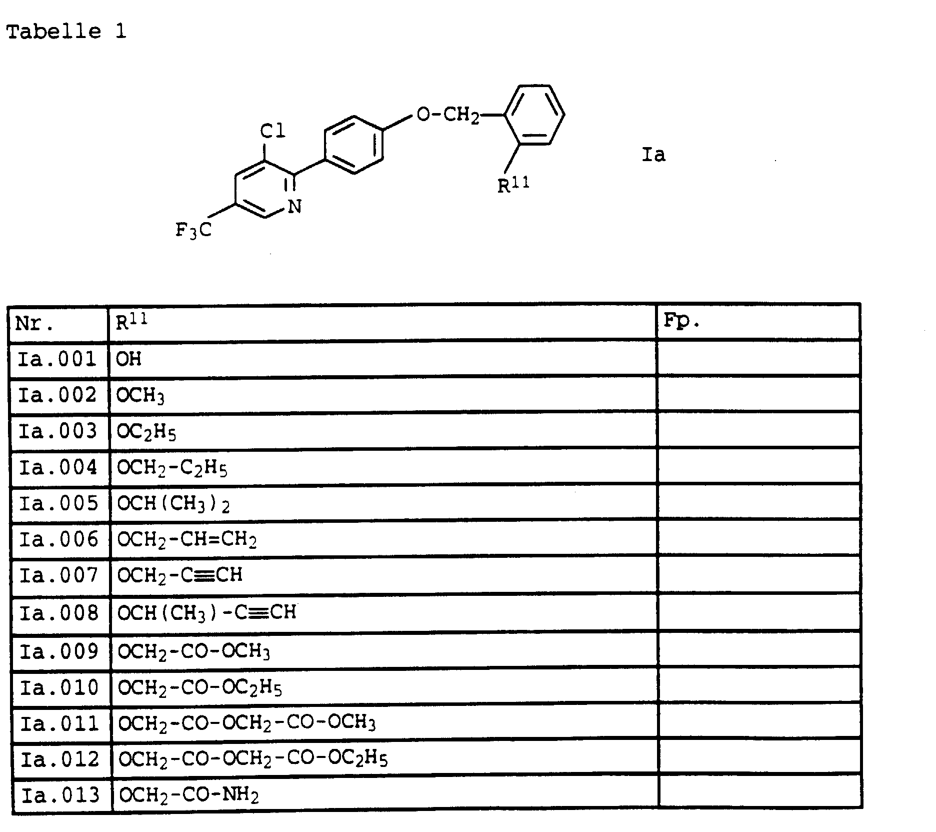

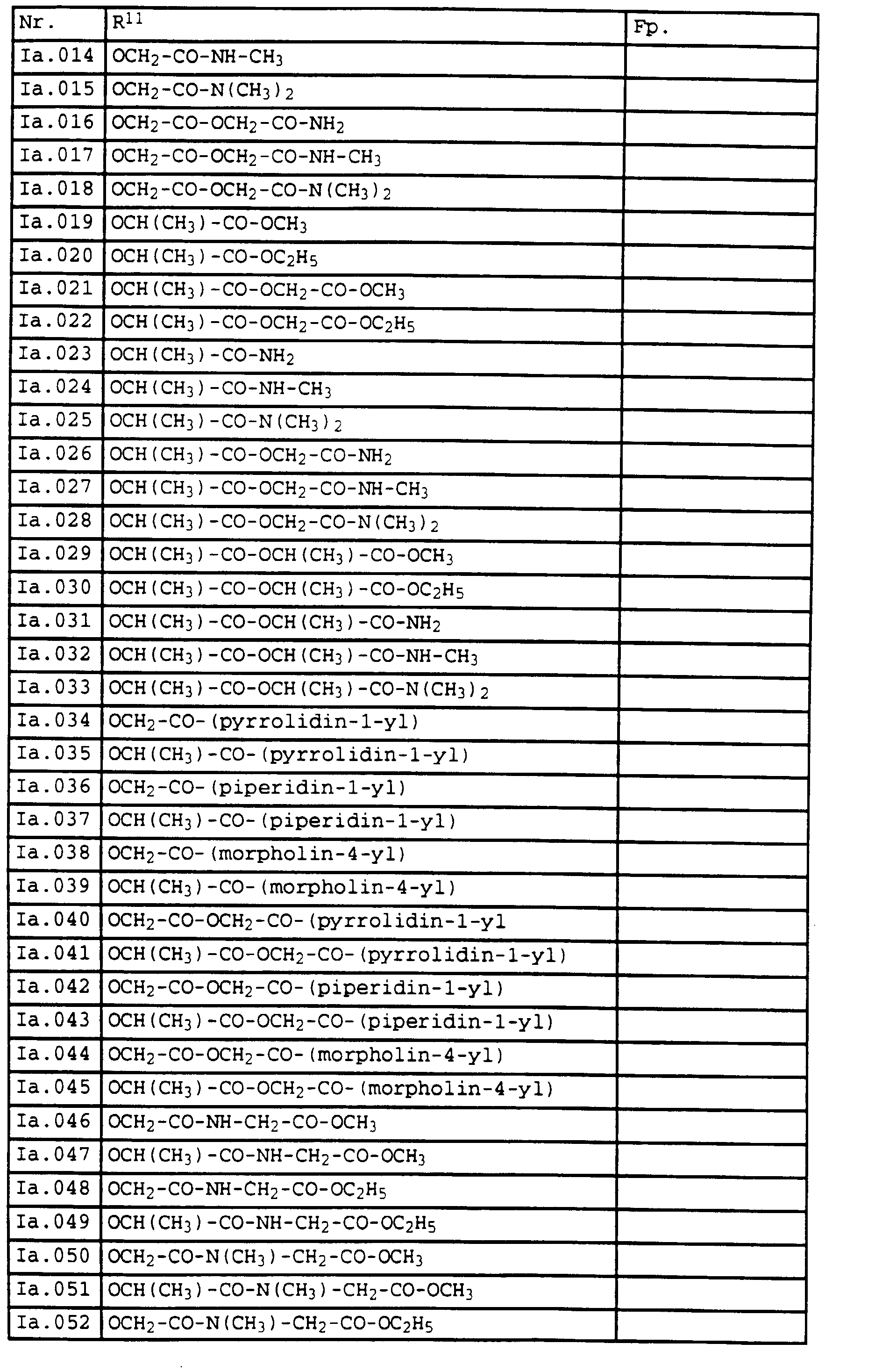

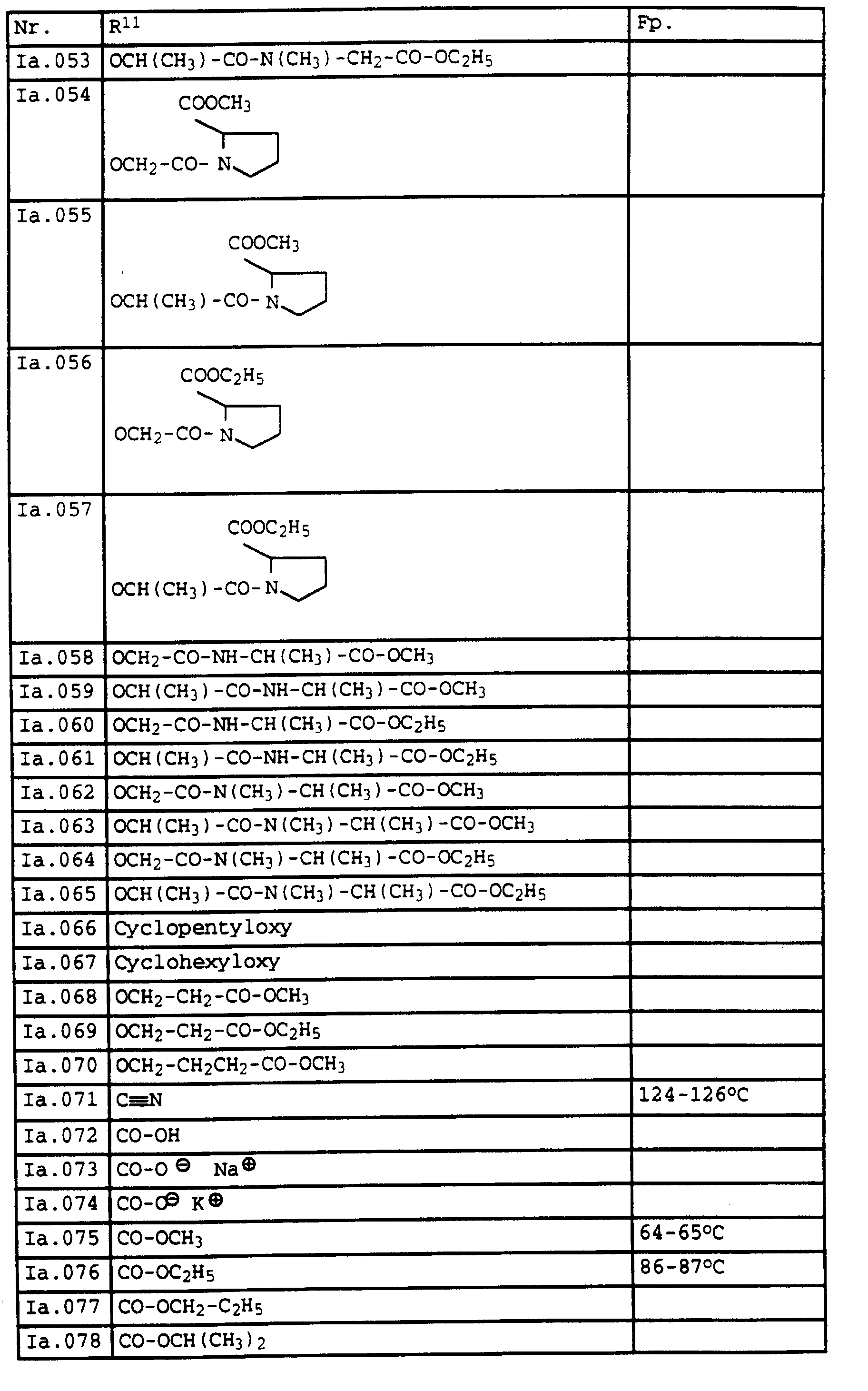

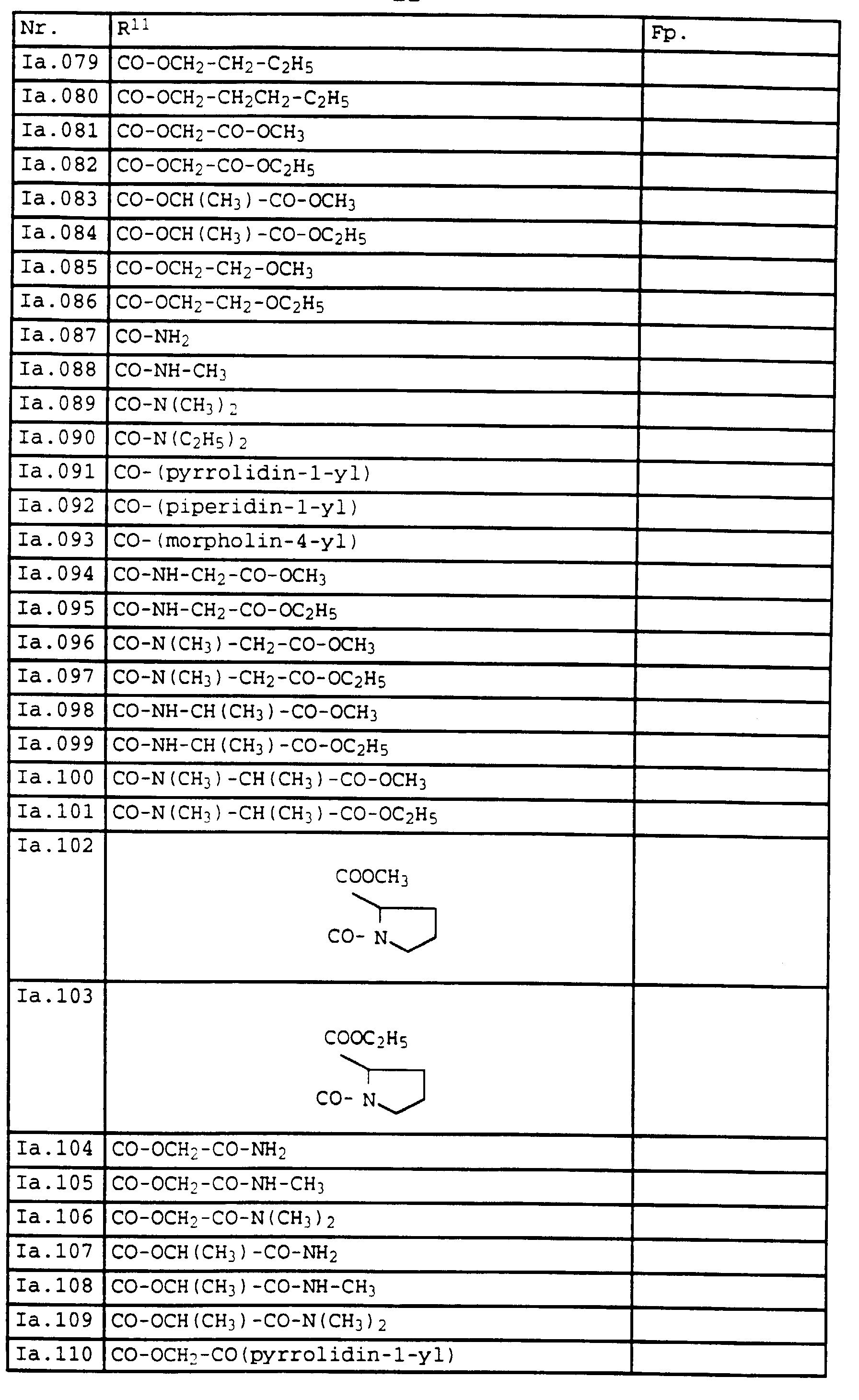

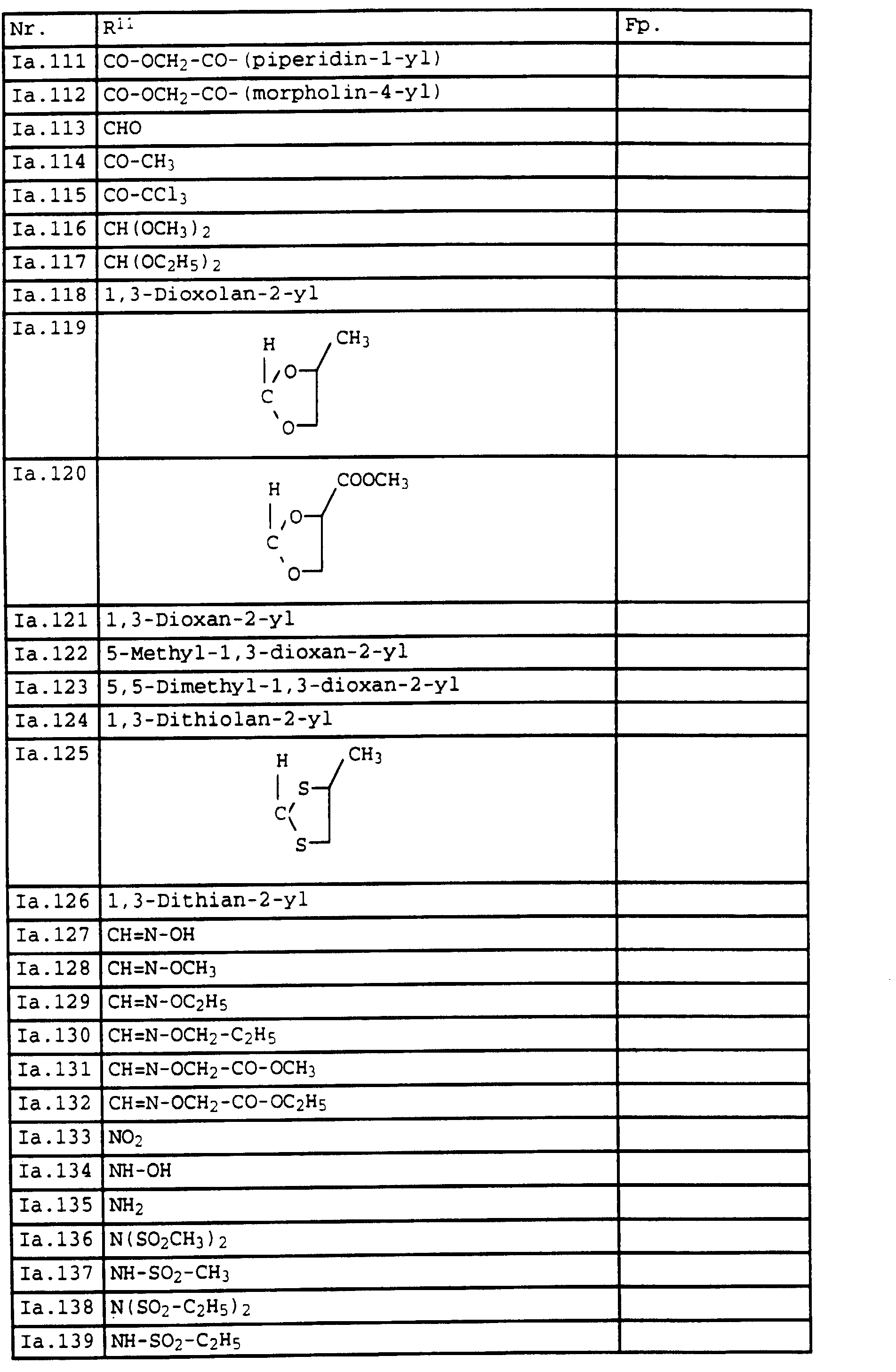

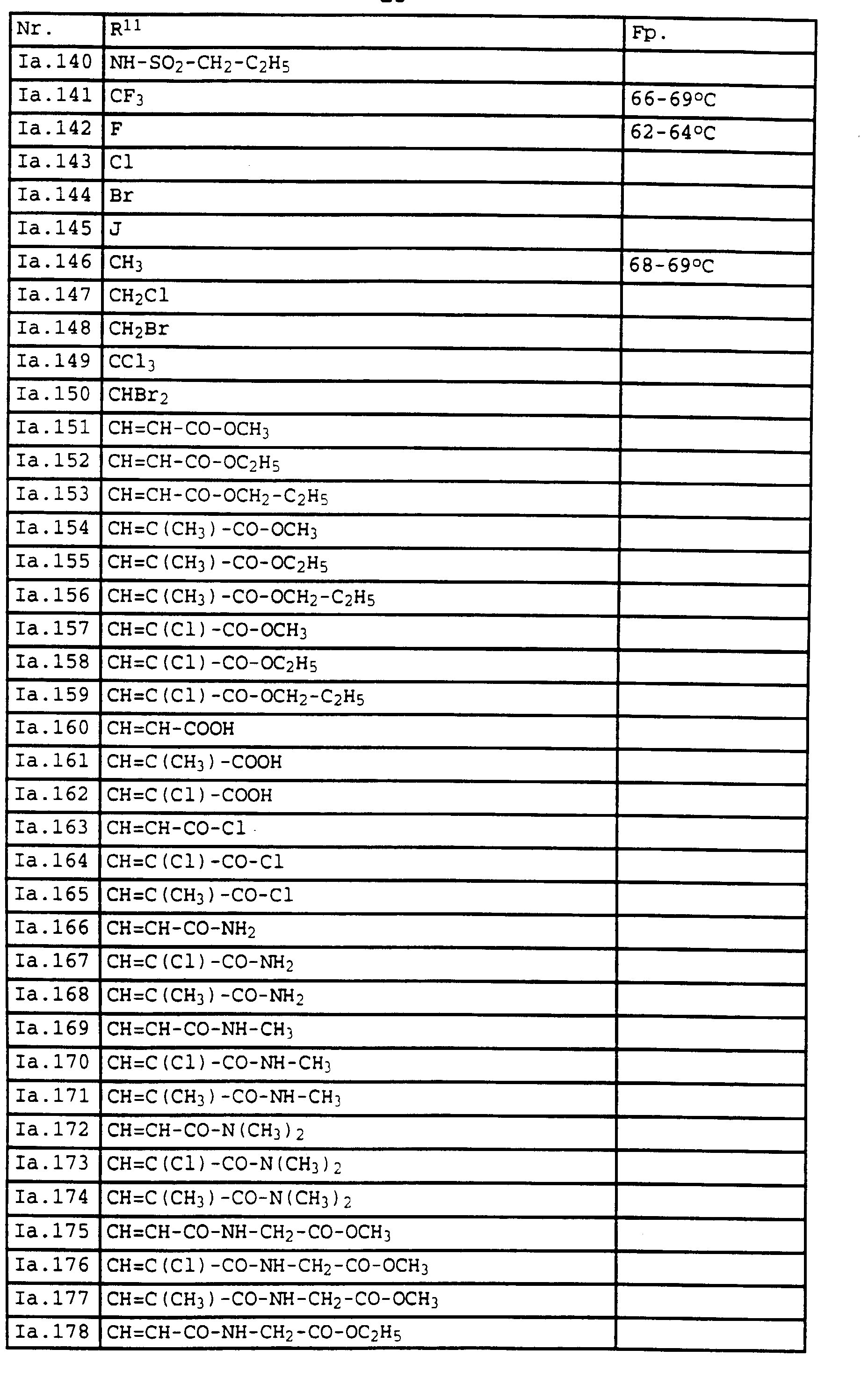

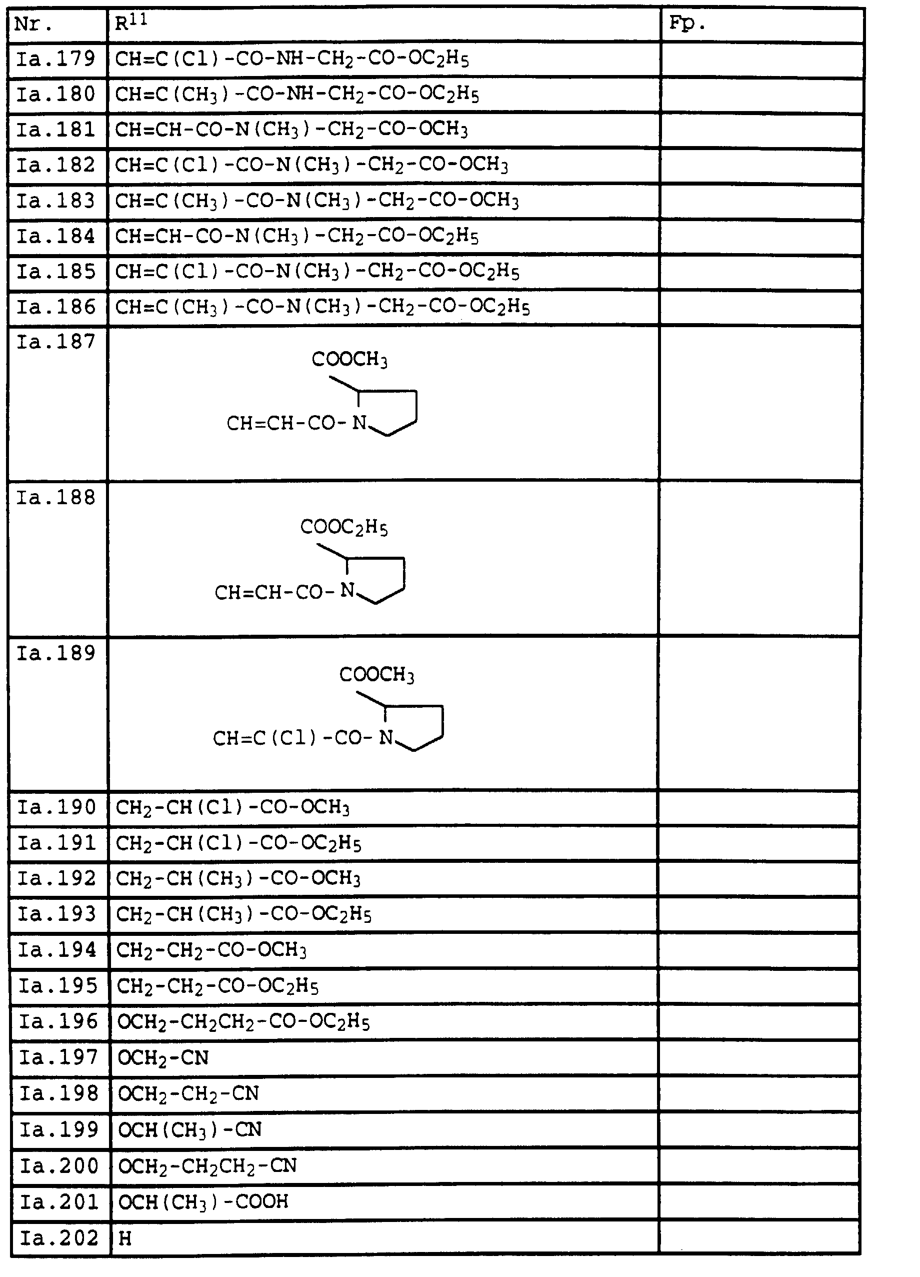

Des weiteren sind die folgenden substituierten 2-Phenylpyridine der Formel I besonders bevorzugt:
- die Verbindungen Ib.001 - Ib.202, die sich von den entsprechenden Verbindungen Ia.001 - Ia.202 lediglich dadurch unterscheiden, daß R⁵ Fluor bedeutet: 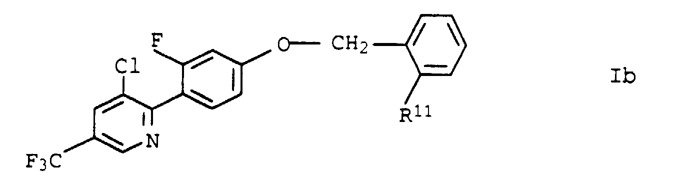
- die Verbindungen Ic.001 - Ic.202, die sich von den entsprechenden Verbindungen Ia.001 - Ia.202 lediglich dadurch unterscheiden, daß R⁹ Methyl bedeutet: 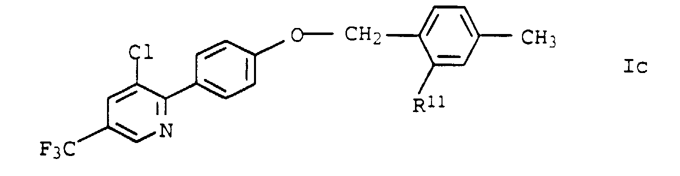
- die Verbindungen Id.001 - Id.202, die sich von den entsprechenden Verbindungen Ia.001 - Ia.202 lediglich dadurch unterscheiden, daß R⁵ Fluor und R⁹ Methyl bedeuten: 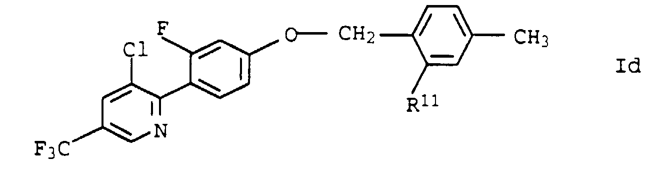
- die Verbindungen Ie.001 - Ie.202, die sich von den entsprechenden Verbindungen Ia.001 - Ia.202 lediglich dadurch unterscheiden, daß R⁹ Chlor bedeutet: 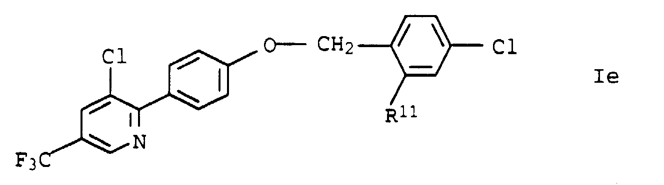
- die Verbindungen If.001 - If.202, die sich von den entsprechenden Verbindungen Ia.001 - Ia.202 lediglich dadurch unterscheiden, daß R⁵ Fluor und R⁹ Chlor bedeuten: 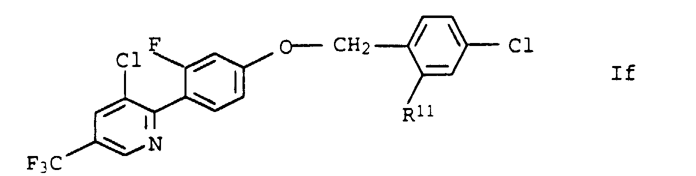
- die Verbindungen Ig.001 - Ig.202, die sich von den entsprechenden Verbindungen Ia.001 - Ia.202 lediglich dadurch unterscheiden, daß R¹⁰ Methyl bedeutet: 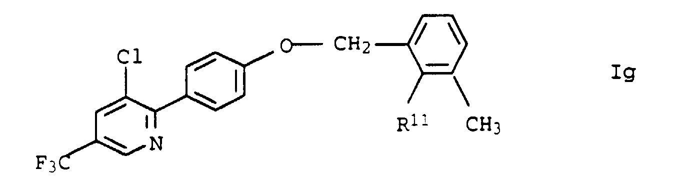
- die Verbindungen Ih.001 - Ih.202, die sich von den entsprechenden Verbindungen Ia.001 - Ia.202 lediglich dadurch unterscheiden, daß R⁵ Fluor und R¹⁰ Methyl bedeuten: 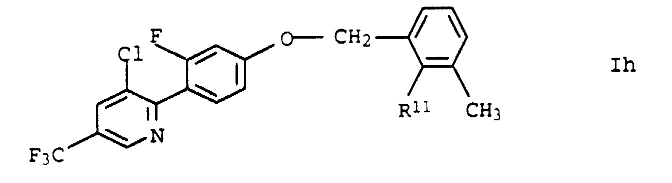
- die Verbindungen Ii.001 - Ii.202, die sich von den entsprechenden Verbindungen Ia.001 - Ia.202 lediglich dadurch unterscheiden, daß R¹⁰ Chlor bedeutet: 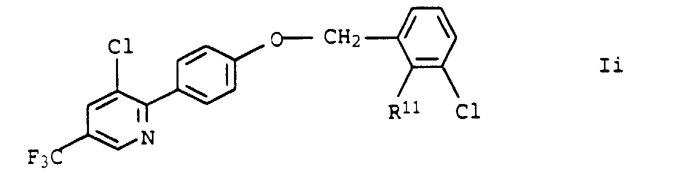
- die Verbindungen Ik.001 - Ik.202, die sich von den entsprechenden Verbindungen Ia.001 - Ia.202 lediglich dadurch unterscheiden, daß R⁵ Fluor und R¹⁰ Chlor bedeuten: 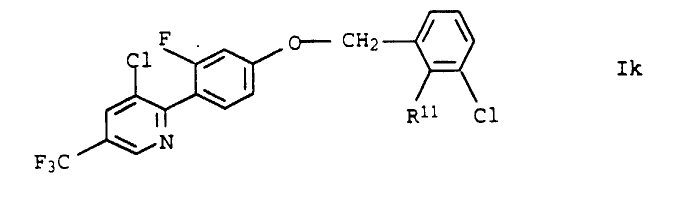
- die Verbindungen Im.001 - Im.202, die sich von den entsprechenden Verbindungen Ia.001 - Ia.202 lediglich dadurch unterscheiden, daß R⁷ Fluor bedeutet:

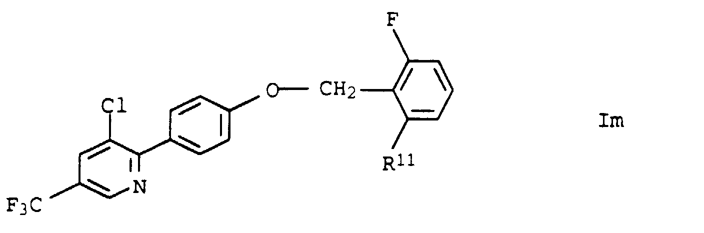

Die substituierten 2-Phenylpyridine der Formel I sind auf verschiedene Weise erhältlich, beispielsweise nach einem der folgenden Verfahren:

### Verfahren A:

Umsetzung von 2-(4-Hydroxyphenyl)pyridinen II mit Benzylhalogeniden oder -mesylaten III auf an sich bekannte Weise {siehe z.B. H. Meerwein in Houben-Weyl, Methoden der organischen Chemie, Bd. VI/3, Teil 3, Seiten 54ff. und A.D. Batcho & W. Leingruber, Org. Synth. 63, 214 (1984)}: 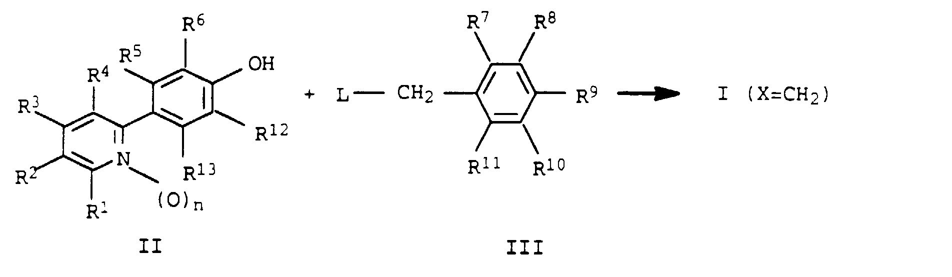 L steht für Chlor, Brom oder Methylsulfonyloxy.

Die Reaktionsführung erfolgt vorzugsweise in einem inerten aprotischen Lösungsmittel wie Dimethylformamid, Dimethylsulfoxid, Acetonitril und Aceton.

In Abhängigkeit von den jeweiligen Substituenten und den Reaktionsbedingungen kann es vorteilhaft sein, in Gegenwart einer in etwa äquivalenten Menge Base, bezogen auf II, zu arbeiten.

Als Basen kommen z.B. Alkalimetallcarbonate wie Natriumhydrogencarbonat und Natriumcarbonat, ferner Stickstoffbasen wie Pyridin und Triethylamin in Betracht.

Die Reaktionstemperatur liegt normalerweise bei 0 bis 100°C.

Üblicherweise werden die Komponenten in etwa stöchiometrischem Verhältnis eingesetzt, jedoch kann ein Überschuß einer der Komponenten, z.B. im Hinblick auf eine möglichst vollständige Umsetzung der anderen Komponente, vorteilhaft sein.

### Verfahren B:

Umsetzung von 2-(4-Hydroxyphenyl)pyridinen II mit Benzoylhalogeniden IV auf an sich bekannte Weise (siehe z.B. H. Henecka in Houben-Weyl, Methoden der organischen Chemie, Bd. VIII, Seiten 543ff. und T.S. Wheeler, Org. Synth. Coll. Vol. IV 1963, Seite 478): 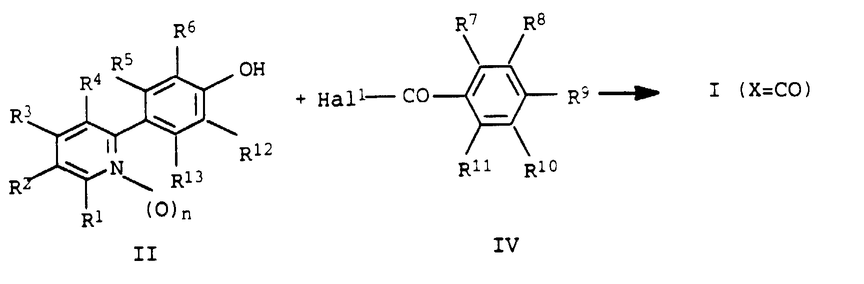 Hal¹ steht für Chlor oder Brom.

Geeignete inerte Lösungsmittel sind insbesondere chlorierte aliphatische Kohlenwasserstoffe wie Chloroform, Dichlormethan, Tetrachlorkohlenstoff und 1,2-Dichlorethan.

In Abhängigkeit von den jeweiligen Substituenten und den Reaktionsbedingungen kann es vorteilhaft sein, in Gegenwart einer Base zu arbeiten.

Bezüglich Base, Reaktionstemperatur und den Mengenverhältnissen gelten die für Verfahren A) gemachten Angaben.

### Verfahren C:

Umsetzung von 2-(4-Hydroxyphenyl)pyridinen II mit substituierten Phthalsäureanhydriden V auf an sich bekannte Weise {siehe z.B. H. Henecka in Houben-Weyl, Methoden der organischen Chemie, Bd. VIII, Seiten 547ff. und P.C. Crofts et al., Org. Prep. Proced. Int. 22 (4), 538-540 (1990)}: 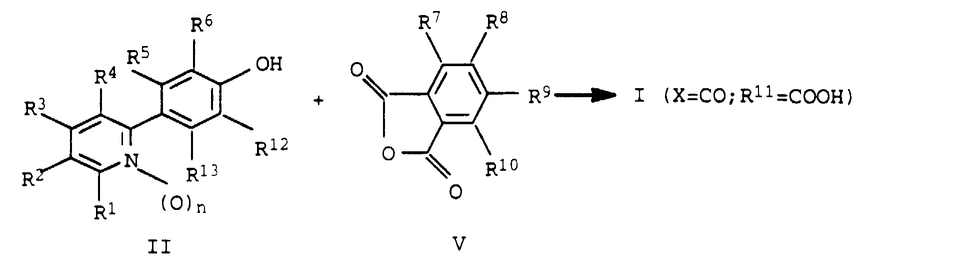 Geeignete Lösungsmittel sind z.B. die bei Verfahren B genannten Chlorkohlenwasserstoffe.

Normalerweise arbeitet man bei einer Reaktionstemperatur von 0 bis 100°C.

Üblicherweise werden die Reaktionspartner in etwa stöchiometrischen Mengen eingesetzt, jedoch besteht auch die Möglichkeit, eine der Komponenten im Überschuß einzusetzen.

### Verfahren D:

Oxidation von substituierten 2-Phenylpyridinen der Formel I, bei denen n Null bedeutet, auf an sich bekannte Weise {vgl. z.B. A. Albini u. S. Pietra, Heterocyclic N-Oxides, CRC-Press Inc., Boca Raton, USA 1991; H.S. Mosher et al., Org. Synth. Coll. Vol. IV 1963, 828; E.C. Taylor et al., Org. Synth. Coll. Vol. IV 1963, 704; T.W. Bell et. al., Org. Synth. 69, 226 (1990)}: 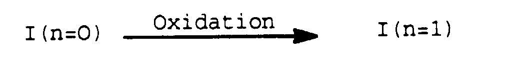 Unter den zur Oxidation des Pyridinrings üblichen Oxidationsmitteln sei beispielhaft auf Peressigsäure, Pertrifluoressigsäure, Perbenzoesäure, m-Chlorperbenzoesäure, Monopermaleinsäure, Magnesiummonoperphthalat, Natriumperborat, Oxone® (enthält Peroxidisulfat), Perwolframsäure und Wasserstoffperoxid verwiesen.

Geeignete Lösungsmittel sind z.B. Wasser, Schwefelsäure, Carbonsäuren wie Essigsäure und Trifluoressigsäure sowie halogenierte Kohlenwasserstoffe wie Dichlormethan und Chloroform.

Normalerweise gelingt die Oxidation bei Temperaturen von 0°C bis Siedetemperatur des Reaktionsgemisches.

Das Oxidationsmittel wird normalerweise in mindestens äuqimolaren Mengen, bezogen auf die Ausgangsverbindung, eingesetzt. In Einzelfällen kann auch ein großer Überschuß an Oxidationsmittel Vorteile bieten.

### Verfahren E:

Umsetzung von 2-Halogenpyridinen VI mit Boronsäuren VII, Boroxinen VIII oder, sofern die Substituenten R¹ bis R¹³ unter den Bedingungen der Grignard-Reaktin inert sind, mit Grignard-Verbindungen IX auf an sich bekannte Weise in Gegenwart einer Base und eines Katalysators (vgl. z.B. DE-A 43 23 916 und die dort zitierte Literatur): 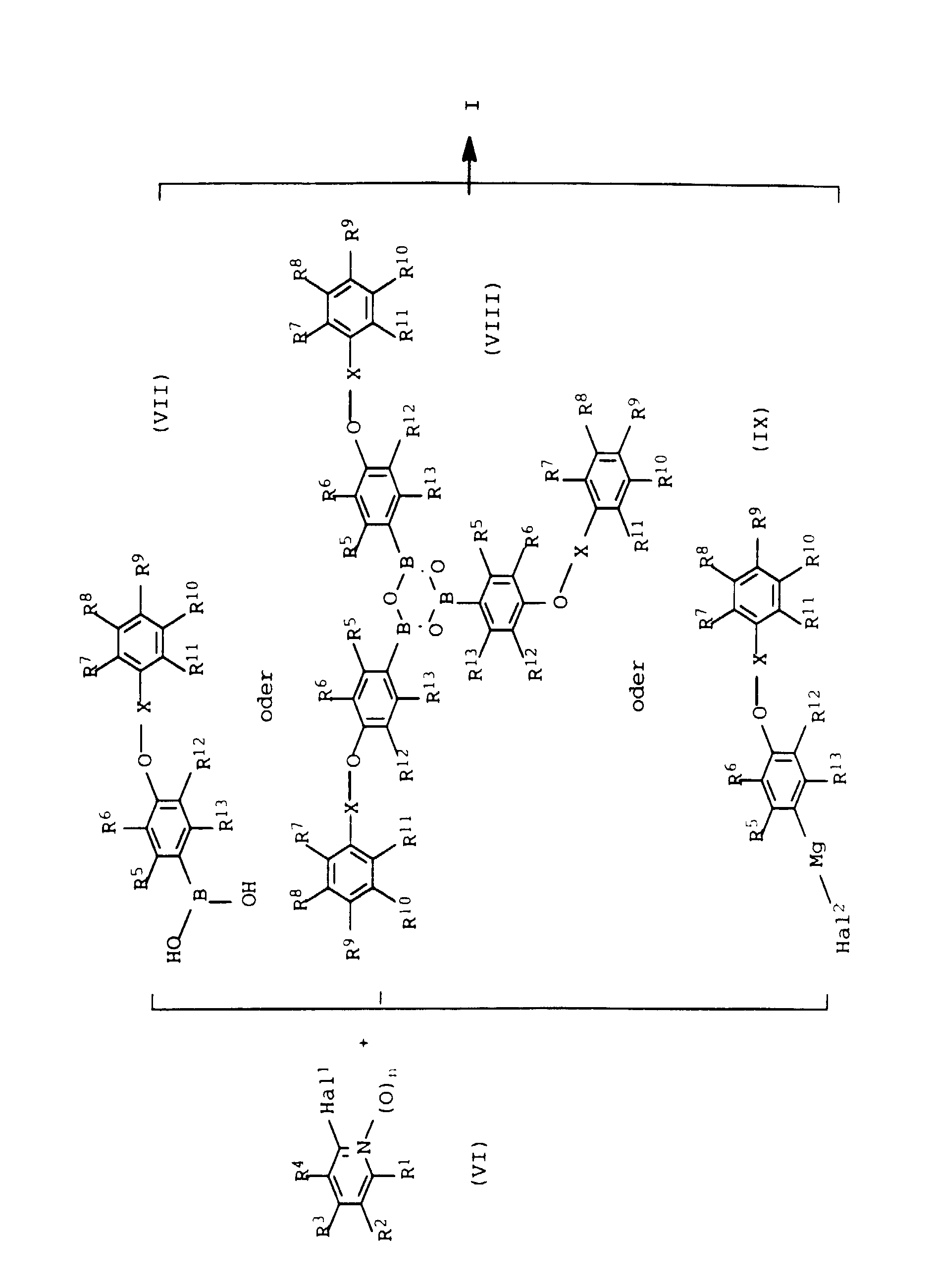
Hal¹ steht für Chlor oder Brom;
Hal² steht für Brom oder Jod.

In der Regel arbeitet man in einem inerten Lösungsmittel(gemisch), beispielsweise in Tetrahydrofuran/Wasser, Dioxan/Wasser, Dimethoxyethan/Wasser, Toluol/Wasser oder in Dimethylformamid.

Als Katalysatoren empfehlen sich Palladium- und Nickelverbindungen wie Tetrakis- (triphenylphosphin)-palladium(0), [1,2-Bis-(diphenylphosphino)-ethan]-palladium(II)-chlorid, [1,1'-Bis-(diphenylphosphino)-ferrocen]-palladium(II)-chlorid, Palladium(II)-acetat und Bis-(triphenylphosphin)-nickel(II)-chlorid. Sie werden bevorzugt in katalytischen Mengen eingesetzt.

Geeignete Basen sind z.B. Alkalimetallcarbonate wie Natriumcarbonat, Alkalimetallhydrogencarbonate wie Natriumhydrogencarbonat, Alkalimetallhydroxide wie Natriumhydroxid, Alkalimetallphosphate wie Kaliumphosphat sowie Alkylamine wie Triethylamin.

Die Reaktion wird allgemein bei 20°C bis zur Siedetemperatur des Reaktionsgemisches vorgenommen.

Das molare Verhältnis von VI zu Base beträgt im allgemeinen 1:1 bis 1:5.

Üblicherweise werden die Ausgangsverbindungen VI und VII, VIII oder IX in etwa äquivalenten Mengen eingesetzt, jedoch kann auch eine der Komponenten im Überschuß eingesetzt werden.

Die 2-Halogenpyridine VI sind bekannt oder auf an sich bekannte Weise erhältlich (vgl. z.B. D. Spitzner in Houben-Weyl, Methoden der Organischen Chemie, Bd. E7b, 286 ff.). Die Boronsäuren VII und Boroxine VIII sind z.B. aus der DE-A 42 36 105, EP-A 569 193 oder der JP-A 05/025 158 bekannt, oder sie sind in an sich bekannter Weise herstellbar (vgl. z.B. M.F. Lappert, Chem. Rev. 56, 959 (1956); K. Torssell in H. Steinberg u. A.L. McCloskey (Ed): Progress in Boron Chemistry, 1, 369 (1964) und R. Köster in Houben-Weyl, Methoder der Organischen Chemie, Bd. 13/3a, 4. Auflage 1982, 617 ff.).

Sofern nicht anders angegeben werden alle vorstehend beschriebenen Verfahren zweckmäßigerweise bei Atmosphärendruck oder unter dem Eigendruck des jeweiligen Reaktionsgemisches vorgenommen.

Die Aufarbeitung der Reaktionsgemische erfolgt in der Regel nach an sich bekannten Methoden, beispielsweise durch Entfernen des Lösungsmittels, Verteilen des Rückstandes in einem Gemisch aus Wasser und einem geeigneten organischen Lösungsmittel und Aufarbeiten der organischen Phase auf das Produkt hin.

Die für die einzelnen Verfahren angegebenen Ausgangsverbindungen sind entweder bekannt oder auf an sich bekannte Weise z. B. auch nach einem der beschriebenen Verfahren, erhältlich.

Die substituierten 2-Phenylpyridine der Formel I können ein oder mehrere Chiralitätszentren enthalten und fallen dann üblicherweise als Enantiomeren- oder Diastereomerengemische an. Die Mischungen können gewünschtenfalls nach den hierfür üblichen Methoden, z.B. mittels Kristallisation oder Chromatographie an einem optisch aktiven Adsorbat, in die weitgehend reinen Isomeren getrennt werden. Reine optisch aktive Isomere lassen sich beispielsweise auch aus entsprechenden optisch aktiven Ausgangsmaterialien herstellen.

Substituierte 2-Phenylpyridine I mit CH-aciden Substituenten lassen sich auf an sich bekannte Weise in ihre Salze, vorzugsweise in ihre Alkalimetallsalze, überführen.

Salze von I, deren Metallion kein Alkalimetallion ist, können durch Umsalzen des entsprechenden Alkalimetallsalzes in üblicher Weise hergestellt werden, ebenso Ammonium-, Phosphonium-, Sulfonium- und Sulfoxoniumsalze mittels Ammoniak, Phosphonium-, Sulfonium- oder Sulfoxoniumhydroxiden.

Diejenigen Verbindungen I, die eine endständige Aminogruppe tragen, können ferner Säureadditionssalze bilden.

Die Verbindungen I/I' und deren landwirtschaftlich brauchbaren Salze eignen sich - sowohl als Isomerengemische als auch in Form der reinen Isomeren - als Herbizide. Die I/I' enthaltenden herbiziden Mittel bekämpfen Pflanzenwuchs auf Nichtkulturflächen sehr gut, besonders bei hohen Aufwandmengen. In Kulturen wie Weizen, Reis, Mais, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf.

In Abhängigkeit von der jeweiligen Applikationsmethode können die Verbindungen I/I' bzw. sie enthaltenden herbiziden Mittel noch in einer weiteren Zahl von Kulturpflanzen zur Beseitigung unerwünschter Pflanzen eingesetzt werden. In Betracht kommen beispielsweise folgende Kulturen: Allium cepa, Ananas comosus, Arachis hypogaea, Asparagus officinalis, Beta vulgaris spp. altissima, Beta vulgaris spp. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spp., Manihot esculenta, Medicago sativa, Musa spp., Nicotiana tabacum (N.rustica), Olea europaea, Oryza sativa , Phaseolus lunatus, Phaseolus vulgaris, Picea abies, Pinus spp., Pisum sativum, Prunus avium, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera und Zea mays.

Darüber hinaus können die Verbindungen I/I' auch in Kulturen, die durch Züchtung einschließlich gentechnischer Methoden gegen die Wirkung von Herbiziden tolerant sind, verwandt werden.

Des weiteren eignen sich die substituierten 2-Phenylpyridine I/I' auch zur Desikkation und/oder Defoliation von Pflanzen.

Als Desikkantien eignen sie sich insbesondere zur Austrocknung der oberirdischen Teile von Kulturpflanzen wie Kartoffel, Raps, Sonnenblume und Sojabohne. Damit wird ein vollständig mechanisches Beernten dieser wichtigen Kulturpflanzen ermöglicht.

Von wirtschaftlichem Interesse ist ferner die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Derselbe Mechanismus, das heißt die Förderung der Ausbildung von Trenngewebe zwischen Frucht- oder Blatt- und Sproßteil der Pflanzen ist auch für ein gut kontrollierbares Entblättern von Nutzpflanzen, insbesondere Baumwolle, wesentlich.

Außerdem führt die Verkürzung des Zeitintervalls, in dem die einzelnen Baumwollpflanzen reif werden, zu einer erhöhten Faserqualität nach der Ernte.

Die Verbindungen I/I' bzw. die sie enthaltenden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Als inerte Hilfsstoffe kommen im wesentlichen in Betracht: Mineralölfraktionen von mittlerem bis hohem Siedepunkt wie Kerosin und Dieselöl, ferner Kohlenteeröle sowie Öle pflanzlichen oder tierischen Ursprungs, aliphatische, cyclische und aromatische Kohlenwasserstoffe, z.B. Paraffine, Tetrahydronaphthalin, alkylierte Naphthaline und deren Derivate, alkylierte Benzole und deren Derivate, Alkohole wie Methanol, Ethanol, Propanol, Butanol und Cyclohexanol, Ketone wie Cyclohexanon, stark polare Lösungsmittel, z.B. Amine wie N-Methylpyrrolidon und Wasser.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe (Adjuvantien) kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylen- oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Konzentrationen der Wirkstoffe I/I' in den anwendungsfertigen Zubereitungen können in weiten Bereichen variiert werden. Im allgemeinen enthalten die Formulierungen von 0,001 bis 98 Gew.-%, vorzugsweise 0,01 bis 95 Gew.-%, Wirkstoff. Die Wirkstoffe werden dabei in einer Reinheit von 90% bis 100%, vorzugsweise 95% bis 100% (nach NMR-Spektrum) eingesetzt.

Die folgenden Formulierungsbeispiele verdeutlichen die Herstellung solcher Zubereitungen:
I. 20 Gewichtsteile der Verbindung Nr. Ia.075 werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100000 Gewichtsteilen Wasser erhält man eine wäßrige Dispersion, die 0,02 Gew.-% des Wirkstoffs enthält.
II. 20 Gewichtsteile der Verbindung Nr. Ia.076 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100000 Gewichtsteilen Wasser erhält man eine wäßrige Dispersion, die 0,02 Gew.-% des Wirkstoffs enthält.
III. 20 Gewichtsteile des Wirkstoffs Nr. Ic.019 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100000 Gewichtsteilen Wasser erhält man eine wäßrige Dispersion, die 0,02 Gew.-% des Wirkstoffs enthält.
IV. 20 Gewichtsteile des Wirkstoffs Nr. Ic.020 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20000 Gewichtsteilen Wasser enthält man eine Spritzbrühe, die 0,1 Gew.-% des Wirkstoffs enthält.
V. 3 Gewichtsteile des Wirkstoffs Nr. Ic.201 werden mit 97 Gewichtsteilen feinteiligem Kaolin vermischt. Man erhält auf diese Weise ein Stäubemittel, das 3 Gew.% des Wirkstoffs enthält.
VI. 20 Gewichtsteile des Wirkstoffs Nr. Ia.071 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.
VII. 1 Gewichtsteil der Verbindung Nr. Ia.141 wird in einer Mischung gelöst, die aus 70 Gewichtsteilen Cyclohexanon, 20 Gewichtsteilen ethoxyliertem Isooctylphenol und 10 Gewichtsteilen ethoxyliertem Ricinusöl besteht. Man erhält ein stabiles Emulsionskonzentrat.
VIII. 1 Gewichtsteil der Verbindung Nr. Ia.146 wird in einer Mischung gelöst, die aus 80 Gewichtsteilen Cyclohexanon und 20 Gewichtsteilen Emulphor EL ¹⁾ besteht. Man erhält ein stabiles Emulsionskonzentrat.

¹⁾ ethoxyliertes Rizinusöl (caster-oil)

Die Applikation der Wirkstoffe I bzw. der herbiziden Mittel kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff I/I' betragen je nach Bekämpfungsziel, Jahreszeit, Zielpflanzen und Wachstumsstadium 0,001 bis 3,0, vorzugsweise 0,01 bis 1 kg/ha aktive Substanz (a.S.).

Zur Verbreiterung des Wirkungsspektrums und zur Erzielung synergistischer Effekte können die substituierten 2-Phenylpyridine I/I' mit zahlreichen Vertretern anderer herbizider oder wachstumsregulierender Wirkstoffgruppen gemischt und gemeinsam ausgebracht werden. Beispielsweise kommen als Mischungspartner 1,2,4-Thiadiazole, 1,3,4-Thiadiazole, Amide, Aminophosphorsäure und deren Derivate, Aminotriazole, Anilide, Aryloxy-/Heteroaryloxyalkansäuren und deren Derivate, Benzoesäure und deren Derivate, Benzothiadiazinone, 2-(Hetaroyl/Aroyl)-1,3-cyclohexandione, Heteroaryl-Aryl-Ketone, Benzylisoxazolidinone, meta-CF₃-Phenylderivate, Carbamate, Chinolincarbonsäure und deren Derivate, Chloracetanilide, Cyclohexan-1,3-dionderivate, Diazine, Dichlorpropionsäure und deren Derivate, Dihydrobenzofurane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5,6-tetrahydrophthalimide, Oxadiazole, Oxirane, Phenole, Aryloxy- und Heteroaryloxyphenoxypropionsäureester, Phenylessigsäure und deren Derivate, 2-Phenylpropionsäure und deren Derivate, Pyrazole, Phenylpyrazole, Pyridazine, Pyridincarbonsäure und deren Derivate, Pyrimidylether, Sulfonamide, Sulfonylharnstoffe, Triazine, Triazinone, Triazolinone, Triazolcarboxamide und Uracile in Betracht.

Außerdem kann es von Nutzen sein, die Verbindungen I/I' allein oder in Kombination mit anderen Herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden. Herstellungsbeispiele

3-Chlor-2-(4-[4-methyl-2-(1-methyl-methoxycarbonylmethoxy)-benzyloxy]-phenyl)-5-trifluormethyl-pyridin (Nr. Ic.019) 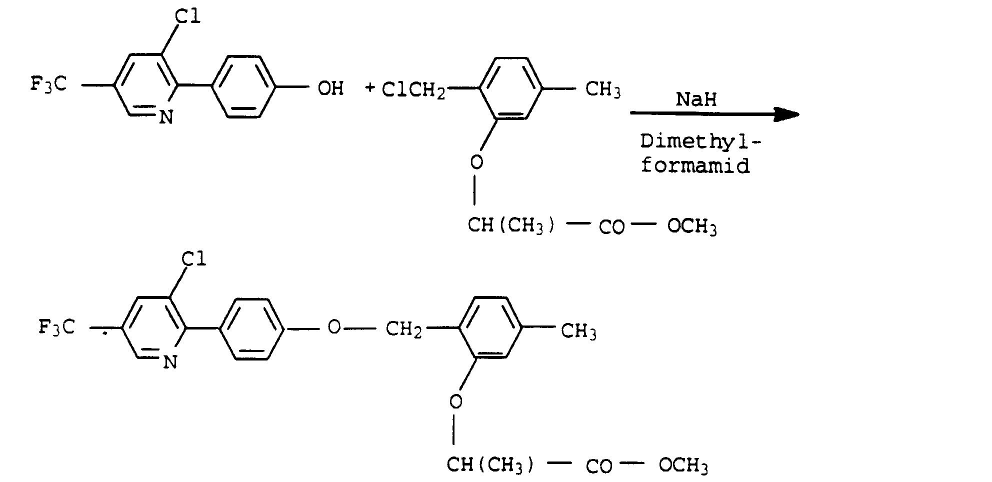 2,5 g einer 80 gew.-%igen Suspension von Natriumhydrid in Mineralöl wurden in 30 ml wasserfreiem Dimethylformamid suspendiert. Zu dieser Suspension tropfte man innerhalb von 30 Minuten eine Lösung von 12,3 g 3-Chlor-2-(4-hydroxyphenyl)-5-trifluormethylpyridin (bekannt aus DE-A 43 23 916) in 150 ml wasserfreiem Dimethylformamid, wonach man noch 10 Minuten rührte. In das Reaktionsgemisch wurde anschließend innerhalb von 10 Minuten eine Lösung von 12,1 g 2-(2-Chlormethyl-5-methyl-phenoxy)-propionsäuremethylester in 50 ml wasserfreiem Dimethylformamid getropft. Danach rührte man acht Stunden bei 80°C und 24 Stunden bei 23°C. Anschließend wurde das Lösungsmittel weitgehend abgedampft. Den Rückstand rührte man in 1,5 l Wasser ein, wonach die wäßrige Phase dreimal mit je 200 ml Methyl-tert.-butylether extrahiert wurde.

Nach Waschen der vereinigten organischen Phasen mit 100 ml Wasser wurde über Natriumsulfat getrocknet und dann eingeengt. Die Reinigung des Rohproduktes erfolgte mittels Chromatographie an Kieselgel mit Cyclohexan/Essigester (4:1) als Elutionsmittel.

Ausbeute: 11,1 g (52 %) weißer Kristalle; Schmelzpunkt: 117-119°C. ¹H-NMR (270 MHz, in CDCl₃): δ [ppm] = 1,65(d,3H), 2,33(s,3H), 3,76(s,3H), 4,85(q,1H), 5,20(d,1H), 5,30(d,1H), 6,60(s,1H), 6,83s(d,1H), 7,13(d,2H), 7,35(d,1H), 7,77(d,2H), 8,00(s,1H), 8,82(s,1H).

In Tabelle 1 und den folgenden Tabellen 2 und 3 sind noch weitere erfindungsgemäße Verbindungen I und auch Verbindungen I' aufgeführt, die auf analoge Weise hergestellt wurden oder herstellbar sind:

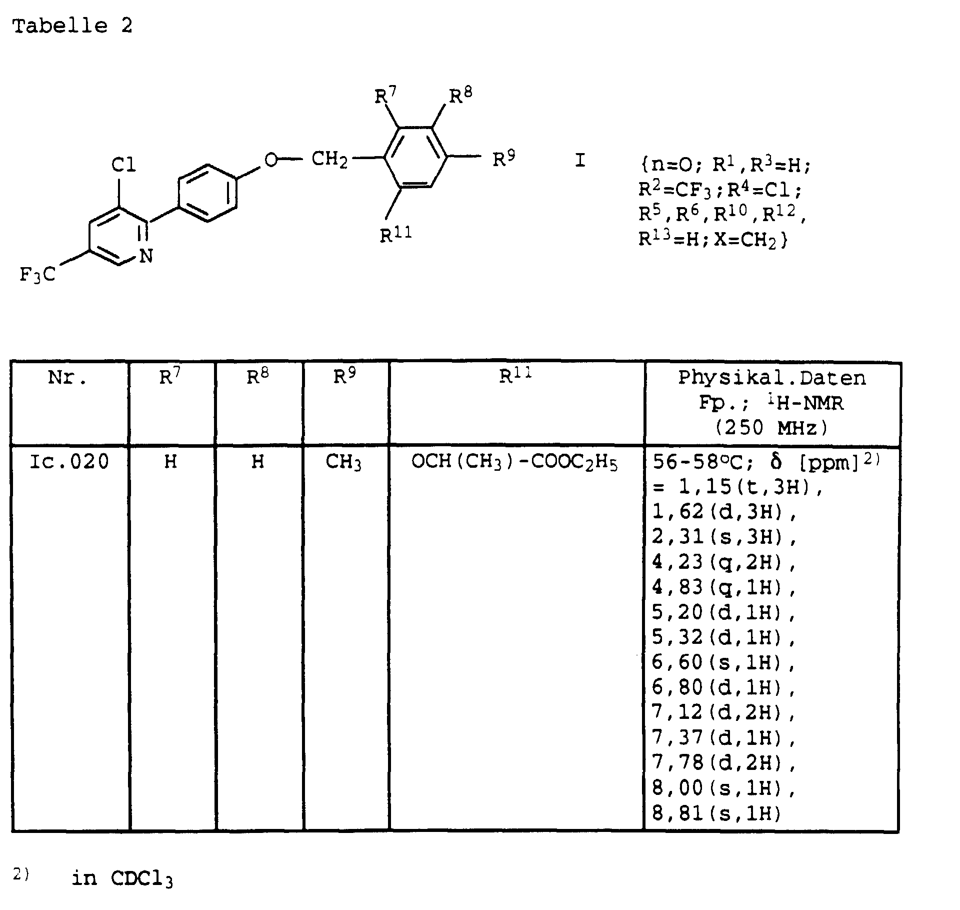

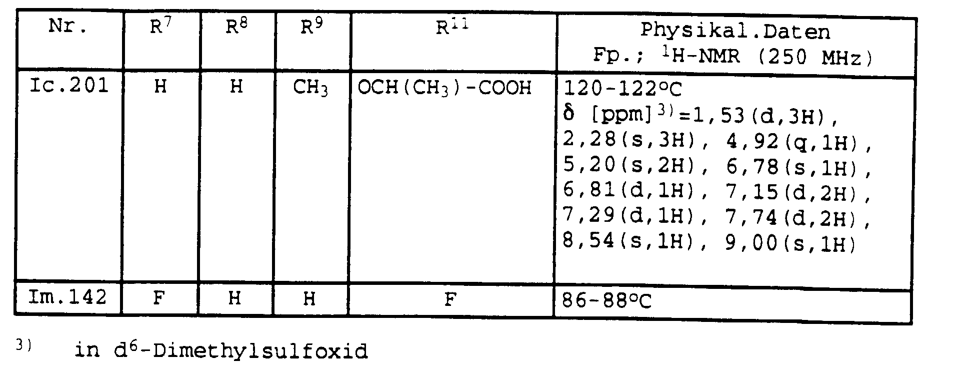

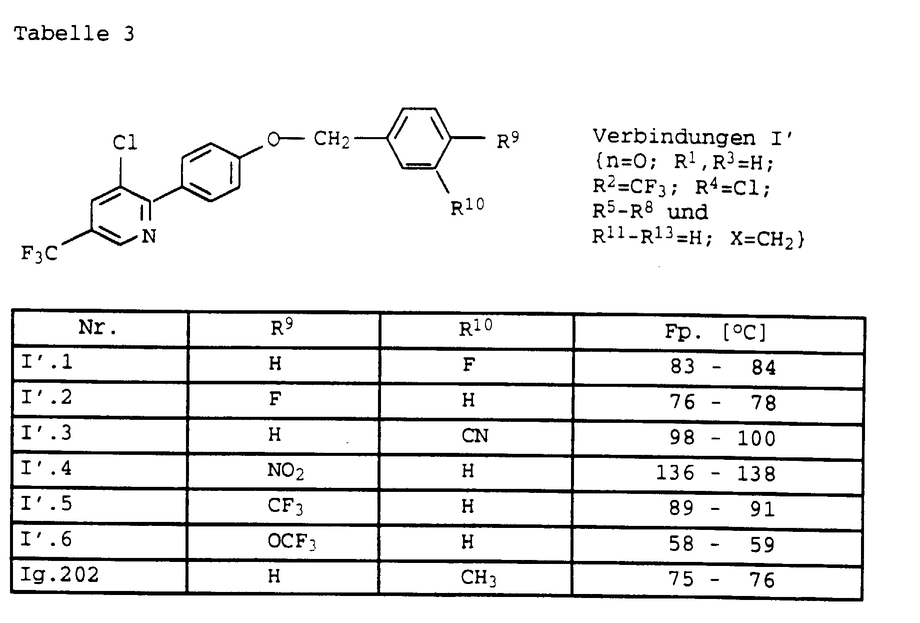

### Anwendungsbeispiele (herbizide Wirksamkeit)

Die herbizide Wirkung der substituierten 2-Phenylpyridine I/I' ließ sich durch die folgenden Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe von 3 bis 15 cm angezogen und erst dann mit den in Wasser suspendierten oder emulgierten Wirkstoffen behandelt. Die Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt. Die Aufwandmenge für die Nachauflaufbehandlung betrug 0,125 oder 0,0625 kg/ha a.S. (aktive Substanz).

Die Pflanzen wurden artenspezifisch bei Temperaturen von 10 - 25°C bzw. 20 - 35°C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name | Englischer Name |
|---|---|---|
| Abutilon theophrasti | Chinesischer Hanf | velvet leaf |
| Ipomoea subspecies | Prunkwindearten | morningglory |
| Solanum nigrum | Schwarzer Nachtschatten | black nightshade |
| Veronica subspecies | Ehrenpreisarten | speedwell |

Bei einer Aufwandmenge von 0,125 oder 0,0625 kg/ha a.S. zeigte die Verbindung Nr. Ic.019 im Nachauflaufverfahren eine sehr gute Wirkung gegen die o.g. Pflanzen.

Anwendungsbeispiele (desikkative/defoliante Wirksamkeit)

Als Testpflanzen dienten junge, 4-blättrige (ohne Keimblätter) Baumwollpflanzen, die unter Gewächshausbedingungen angezogen wurden (rel. Luftfeuchtigkeit 50 bis 70 %; Tag-/Nachttemperatur 27/20°C).

Die jungen Baumwollpflanzen wurden tropfnaß mit wässrigen Aufbereitungen der Wirkstoffe (unter Zusatz von 0,15 Gew.-% des Fettalkoholalkoxylats Plurafac LF 700, bezogen auf die Spritzbrühe) blattbehandelt. Die ausgebrachte Wassermenge betrug umgerechnet 1000 l/ha. Nach 13 Tagen wurde die Anzahl der abgeworfenen Blätter und der Grad der Entblätterung in % bestimmt.

Bei den unbehandelten Kontrollpflanzen trat kein Blattfall auf.

## Patentansprüche

1. Substituierte 2-Phenylpyridine der allgemeinen Formel I 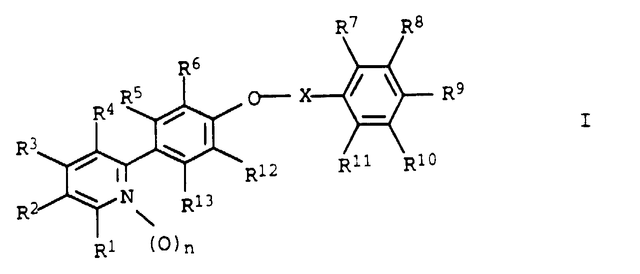 in der die Variablen folgende Bedeutung haben:
n 0 oder 1;
R¹, R³, R⁴ unabhängig voneinander
Wasserstoff, Halogen, C₁-C₄-Alkyl, C₁-C₄-Halogenalkyl, Hydroxy, C₁-C₄-Alkoxy, C₁-C₄-Halogenalkoxy, Nitro, Amino, C₁-C₄-Alkylamino, Di-(C₁-C₄-alkyl)-amino, Mercapto, C₁-C₄-Alkylthio, C₁-C₄-Halogenalkylthio, Cyano, Hydroxycarbonyl, (C₁-C₄-Alkoxy)carbonyl, Aminocarbonyl, C₁-C₄-Alkyl-aminocarbonyl oder Di-(C₁-C₄-alkyl)-aminocarbonyl;
R² Wasserstoff, Halogen, C₁-C₄-Alkyl, C₁-C₄-Halogenalkyl, Cyano, Nitro, Amino, Hydroxy, C₁-C₄-Halogenalkoxy, Mercapto, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl oder C₁-C₄-Halogenalkylsulfonyl;
R⁵ Wasserstoff, Halogen oder Cyano;
R⁶ und R¹³ unabhängig voneinander Wasserstoff oder Halogen;
X Methylen oder Carbonyl;
R⁷, R⁸, R⁹, R¹⁰ unabhängig voneinander Wasserstoff, Nitro, Cyano, Halogen, C₁-C₄-Alkyl, C₁-C₄-Halogenalkyl, C₁-C₄-Alkoxy oder C₁-C₄-Halogenalkoxy;
R¹¹ Wasserstoff, Halogen, C₁-C₄-Alkyl, C₁-C₄-Halogenalkyl, C₁-C₄-Hydroxyalkyl, C₁-C₄-Alkoxy-C₁-C₄-alkyl, C₃-C₆-Cycloalkyl, C₂-C₄-Alkenyl, C₂-C₄-Alkinyl, Hydroxycarbonyl-C₁-C₄-alkyl, Hydroxycarbonyl-C₂-C₄-alkenyl, Hydroxycarbonyl-C₂-C₄-alkinyl, (C₁-C₄-Alkoxy)carbonyl-C₁-C₄-alkyl, (C₁-C₄-Alkoxy)carbonyl-C₂-C₄-alkenyl, (C₁-C₄-Alkoxy)carbonyl-C₂-C₄-alkinyl, (C₁-C₄-Alkoxy)carbonyl-C₂-C₄-halogenalkyl, (C₁-C₄-Alkoxy)carbonyl-C₂-C₄-halogenalkenyl, Aminocarbonyl-C₁-C₄-alkyl, Aminocarbonyl-C₂-C₄-alkenyl, Aminocarbonyl-C₂-C₄-alkinyl, C₁-C₄-Alkylaminocarbonyl-C₁-C₄-alkyl, C₁-C₄-Alkylaminocarbonyl-C₂-C₄-alkenyl, C₁-C₄-Alkylaminocarbonyl-C₂-C₄-alkinyl, Di-(C₁-C₄-alkyl)-aminocarbonyl-C₁-C₄-alkyl, Di-(C₁-C₄-alkyl)-aminocarbonyl-C₂-C₄-alkenyl, Di-(C₁-C₄-alkyl)-aminocarbonyl-C₂-C₄-alkinyl, Aminocarbonyl-C₁-C₄-halogenalkyl, Aminocarbonyl-C₂-C₄-halogenalkenyl, C₁-C₄-Alkylaminocarbonyl-C₁-C₄-halogenalkyl, C₁-C₄-Alkylaminocarbonyl-C₂-C₄-halogenalkenyl, Di-(C₁-C₄-alkyl)-aminocarbonyl-C₂-C₄-halogenalkyl, Di-(C₁-C₄-alkyl)-aminocarbonyl-C₂-C₄-halogenalkenyl, (Hydroxycarbonyl-C₁-C₄-alkyl)-aminocarbonyl-C₂-C₄-alkenyl, (Hydroxycarbonyl-C₁-C₄-alkyl)-aminocarbonyl-C₂-C₄-halogenalkenyl, N-(Hydroxycarbonyl-C₁-C₄-alkyl)-N-(C₁-C₄-alkyl)-aminocarbonyl-C₂-C₄-alkenyl, N-(Hydroxycarbonyl-C₁-C₄-alkyl)-N-(C₁-C₄-alkyl)-aminocarbonyl-C₂-C₄-halogenalkenyl, [(C₁-C₄-Alkoxy)carbonyl-C₁-C₄-alkyl]-aminocarbonyl-C₂-C₄-alkenyl, [(C₁-C₄-Alkoxy)carbonyl-C₁-C₄-alkyl]-aminocarbonyl-C₂-C₄-halogenalkenyl, N-[(C₁-C₄-Alkoxy)carbonyl-C₁-C₄-alkyl]-N-(C₁-C₄-alkyl)-aminocarbonyl-C₂-C₄-alkenyl, N-[(C₁-C₄-Alkoxy)carbonyl-C₁-C₄-alkyl]-N-(C₁-C₄-alkyl)-aminocarbonyl-C₂-C₄-halogenalkenyl, 2-[(C₁-C₄-Alkoxy)carbonyl]-pyrrolidin-1-yl-carbonyl-C₂-C₄-alkenyl, 2-[(C₂-C₄-Alkoxy)carbonyl]-pyrrolidin-1-yl-carbonyl-C₂-C₄-halogenalkenyl, Formyl, (C₁-C₄-Alkyl)carbonyl, (C₁-C₄-Halogenalkyl)carbonyl, Hydroxyimino-C₁-C₄-alkyl, C₁-C₄-Alkoxyimino-C₁-C₄-alkyl, 1,1-Di-(C₁-C₄-alkoxy)-C₁-C₄-alkyl, 1,1-Di-(C₁-C₄-alkylthio)-C₁-C₄-alkyl,
Nitro, Hydroxylamino, Amino, C₁-C₄-Alkylamino, C₃-C₆-Cycloalkylamino, Di-(C₁-C₄-alkyl)-amino, (C₁-C₄-Alkyl)carbonylamino, (C₁-C₄-Halogenalkyl)carbonylamino, C₁-C₄-Alkylsulfonylamino, Di-(C₁-C₄-alkylsulfonyl)-amino, C₁-C₄-Halogenalkylsulfonylamino, Hydroxy, C₁-C₄-Alkoxy, C₁-C₄-Halogenalkoxy, Cyano-C₁-C₄-alkoxy, C₁-C₄-Alkoxy-C₁-C₄-alkoxy, C₁-C₄-Alkylthio-C₁-C₄-alkoxy, C₃-C₆-Cycloalkoxy, C₂-C₄-Alkenyloxy, C₂-C₄-Alkinyloxy, Hydroxycarbonyl-C₁-C₄-alkoxy, (C₁-C₆-Alkoxy)carbonyl-C₁-C₄-alkoxy, C₁-C₄-Alkoxy-(C₁-C₄-alkoxy)carbonyl-C₁-C₄-alkoxy, (C₁-C₄-Alkoxy)carbonyl-(C₁-C₄-alkoxy)carbonyl-C₁-C₄-alkoxy, Aminocarbonyl-C₁-C₄-alkoxy, C₁-C₄-Alkylaminocarbonyl-C₁-C₄-alkoxy, Di-(C₁-C₄-alkyl)-aminocarbonyl-C₁-C₄-alkoxy, 1-Pyrrolidinyl-carbonyl-C₁-C₄-alkoxy, 1-Piperidinyl-carbonyl-C₁-C₄-alkoxy, 4-Morpholinyl-carbonyl-C₁-C₄-alkoxy, Aminocarbonyl-(C₁-C₄-alkoxy)carbonyl-C₁-C₄-alkoxy, (C₁-C₄-Alkyl)aminocarbonyl-(C₁-C₄-alkoxy)carbonyl-C₁-C₄-alkoxy, Di-(C₁-C₄-alkyl)-aminocarbonyl-(C₁-C₄-alkoxy)carbonyl-C₁-C₄-alkoxy, 1-Pyrrolidinyl-carbonyl-(C₁-C₄-alkoxy)carbonyl-C₁-C₄-alkoxy, 1-Piperidinyl-carbonyl-(C₁-C₄-alkoxy)carbonyl-C₁-C₄-alkoxy, 4-Morpholinyl-carbonyl-(C₁-C₄-alkoxy)carbonyl-C₁-C₄-alkoxy, [(C₁-C₄-Alkoxy)carbonyl-C₁-C₄-alkyl]-aminocarbonyl-C₁-C₄-alkoxy, N-[(C₁-C₄-Alkoxy)carbonyl-C₁-C₄-alkyl]-N-(C₁-C₄-alkyl)-aminocarbonyl-C₁-C₄-alkoxy, (2-[(C₁-C₄-Alkoxy)carbonyl]-pyrrolidin-1-yl]-carbonyl-C₁-C₄-alkoxy, Cyano, Hydroxycarbonyl, COCl, (C₁-C₄-Alkoxy)carbonyl, (C₁-C₄-Halogenalkoxy)carbonyl, C₁-C₄-Alkoxy-(C₁-C₄-alkoxy)carbonyl, Aminocarbonyl, C₁-C₄-Alkylaminocarbonyl, Di-(C₁-C₄-alkyl)-aminocarbonyl, C₃-C₆-Cycloalkylaminocarbonyl, 1-Pyrrolidinylcarbonyl, 1-Piperidinylcarbonyl, 4-Morpholinylcarbonyl, Hydroxycarbonyl-C₁-C₄-alkylaminocarbonyl, N-(Hydroxycarbonyl-C₁-C₄-alkyl)-N-(C₁-C₄-alkyl)-aminocarbonyl, (C₁-C₄-Alkoxy)carbonyl-C₁-C₄-alkylaminocarbonyl, N-[(C₁-C₄-Alkoxy)carbonyl-C₁-C₄-alkyl]-N-(C₁-C₄-alkyl)-aminocarbonyl, 2-Hydroxycarbonyl-pyrrolidin-1-yl-carbonyl, 2-[(C₁-C₄-Alkoxy)carbonyl]-pyrrolidin-1-yl-carbonyl oder eine Gruppe 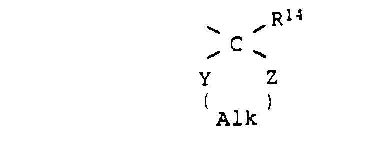
wobei
Y und Z unabhängig voneinander für Sauerstoff oder Schwefel,
R¹⁴ für Wasserstoff, C₁-C₄-Alkyl oder (C₁-C₄-Alkoxy)carbonyl und
Alk für eine Ethylen- oder Trimethylenkette, bei denen ein oder mehrere Wasserstoffatome jeweils durch C₁-C₄-Alkyl oder (C₁-C₄-Alkoxy)carbonyl ersetzt sein können,
stehen;
R¹² Wasserstoff, Nitro, Amino, Hydroxylamino, C₁-C₄-Alkylsulfonylamino, Di-(C₁-C₄-alkylsulfonyl)-amino, C₁-C₄-Halogenalkylsulfonylamino, (C₁-C₄-Alkoxy)carbonyl-C₁-C₄-halogenalkyl oder (C₁-C₄-Alkoxy)carbonyl-C₂-C₄-halogenalkenyl;
mit der Maßgabe, daß R⁵, R⁷, R¹¹ und R¹³ nicht alle gleichzeitig Wasserstoff bedeuten oder R⁷ und R⁸ oder R¹⁰ und R¹¹ nicht gleichzeitig Fluor bedeuten,
sowie die landwirtschaftlich brauchbaren Salze der Verbindungen I, sofern diese existieren.

2. Substituierte 2-Phenylpyridine der Formel I nach Anspruch 1, wobei R¹ und R³ für Wasserstoff, R² für C₁-C₄-Halogenalkyl und R⁴ für Halogen stehen.

3. Verwendung von substituierten 2-Phenylpyridinen der Formel I' und deren landwirtschaftlich brauchbaren Salze, wobei I' der Definition von I gemäß Anspruch 1, jedoch ohne die Maßgabe entspricht, als Herbizide oder zur Desikkation und/oder Defoliation von Pflanzen.

4. Herbizides Mittel, enthaltend eine herbizid wirksame Menge mindestens eines substituierten 2-Phenylpyridins der Formel I oder eines landwirtschaftlich brauchbaren Salzes von I, und mindestens einen inerten flüssigen und/oder festen Trägerstoff sowie gewünschtenfalls mindestens einen oberflächenaktiven Stoff.

5. Mittel zur Desikkation und/oder Defoliation von Pflanzen, enthaltend eine desikkant und/oder defoliant wirksame Menge mindestens eines substituierten 2-Phenylpyridins der Formel I oder eines landwirtschaftlich brauchbaren Salzes von I, und mindestens einen inerten flüssigen und/oder festen Trägerstoff sowie gewünschtenfalls mindestens einen oberflächenaktiven Stoff.

6. Verfahren zur Herstellung von herbizid wirksamen Mitteln, dadurch gekennzeichnet, daß man eine herbizid wirksame Menge mindestens eines substituierten 2-Phenylpyridins der Formel I oder eines landwirtschaftlich brauchbaren Salzes von I, und mindestens einen inerten flüssigen und/oder festen Trägerstoff sowie gewünschtenfalls mindestens einen oberflächenaktiven Stoff mischt.

7. Verfahren zur Herstellung von desiccant und/oder defoliant wirksamen Mitteln, dadurch gekennzeichnet, daß man eine desiccant und/oder defoliant wirksame Menge mindestens eines substituierten 2-Phenylpyridins der Formel I oder eines landwirtschaftlich brauchbaren Salzes von I, und mindestens einen inerten flüssigen und/oder festen Trägerstoff sowie gewünschtenfalls mindestens einen oberflächenaktiven Stoff mischt.

8. Verfahren zur Bekämpfung von unerwünschtem Pflanzenwuchs, dadurch gekennzeichnet, daß man eine herbizid wirksame Menge mindestens eines substituierten 2-Phenylpyridins der Formel I' oder eines landwirtschaftlich brauchbaren Salzes von I', wobei I' der Definition von I gemäß Anspruch 1, jedoch ohne die Maßgabe entspricht, auf Pflanzen, deren Lebensraum oder auf Saatgut einwirken läßt.

9. Verfahren zur Desiccation und/oder Defoliation von Pflanzen, dadurch gekennzeichnet, daß man eine desiccant und/oder defoliant wirksame Menge mindestens eines substituierten 2-Phenylpyridins der Formel I', oder eines landwirtschaftlich brauchbaren Salzes von I', wobei I' der Definition von I gemäß Anspruch 1, jedoch ohne die Maßgabe entspricht, auf Pflanzen einwirken läßt.

10. Verfahren nach Anspruch 9, dadurch gekennzeichnet, daß man Baumwolle behandelt.

11. Verfahren zur Herstellung von substituierten 2-Phenylpyridinen der Formel I gemäß Anspruch 1, bei denen X Methylen bedeutet, dadurch gekennzeichnet, daß man ein 2-(4-Hydroxyphenyl)pyridin der Formel II 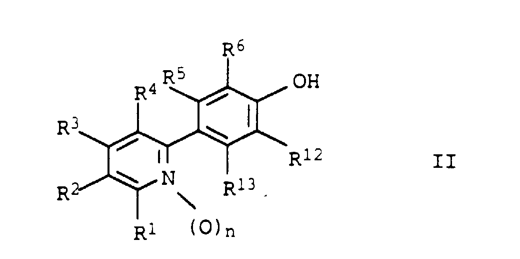 wobei die Variablen die in Anspruch 1 angegebenen Bedeutungen haben,
auf an sich bekannte Weise in einem inerten Lösungs- oder Verdünnungsmittel, gewünschtenfalls in Gegenwart einer Base, mit einem Benzylhalogenid oder -mesylat der Formel III 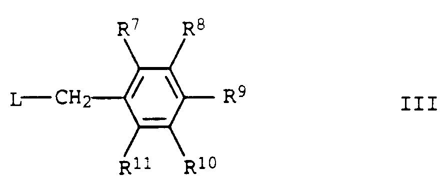 wobei L für Chlor, Brom oder Methylsulfonyloxy steht, umsetzt.

12. Verfahren zur Herstellung von substituierten 2-Phenylpyridinen der Formel I gemäß Anspruch 1, bei denen X Carbonyl bedeutet, dadurch gekennzeichnet, daß man ein 2-(4-Hydroxyphenyl)pyridin der Formel II 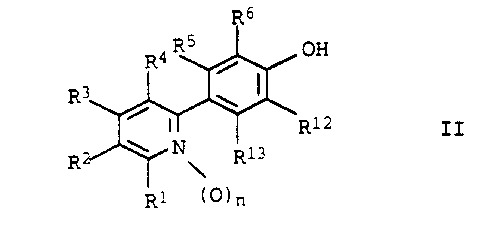 wobei die Variablen die in Anspruch 1 angegebenen Bedeutungen haben,
auf an sich bekannte Weise in einem inerten Lösungs- oder Verdünnungsmittel, gewunschtenfalls in Gegenwart einer Base, mit einem Benzoylhalogenid der Formel IV 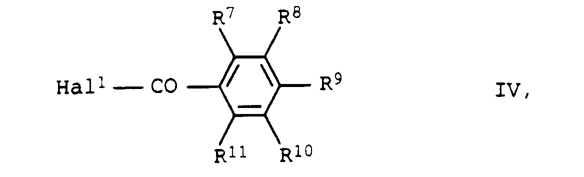 wobei Hal¹ für Chlor oder Brom steht, umsetzt.

13. Verfahren zur Herstellung von substituierten 2-Phenylpyridinen der Formel I gemäß Anspruch 1, bei denen X Carbonyl und R¹¹ Hydroxycarbonyl bedeuten, dadurch gekennzeichnet, daß man ein 2-(4-Hydroxyphenyl)pyridin der Formel II 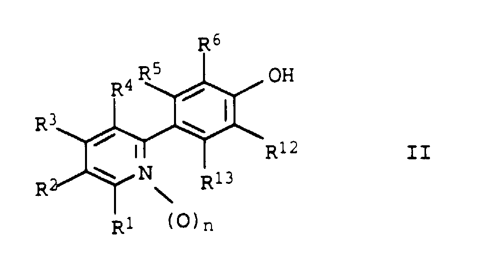 wobei die Variablen die in Anspruch 1 angegebenen Bedeutungen haben,
auf an sich bekannte Weise in einem inerten Lösungs- oder Verdünnungsmittel mit einem substituierten Phthalsäureanhydrid der Formel V 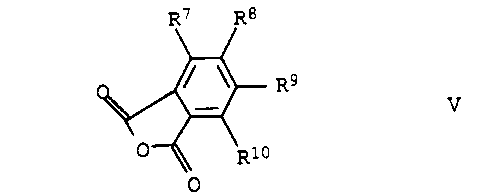 umsetzt.

14. Verfahren zur Herstellung von substituierten 2-Phenylpyridinen der Formel I gemäß Anspruch 1, bei denen n 1 bedeutet, dadurch gekennzeichnet, daß man das entsprechende substituierte 2-Phenylpyridin, bei dem n für 0 steht, auf an sich bekannte Weise oxidiert.

15. Verwendung der 2-(4-Hydroxyphenyl)pyridine der Formel II, wobei die Variablen die in Anspruch 1 angegebenen Bedeutungen haben, zur Herstellung von substituierten 2-Phenylpyridinen der Formel I gemäß Anspruch 1.

16. Verfahren zur Herstellung von substituierten 2-Phenylpyridinen der Formel I gemäß Anspruch 1, dadurch gekennzeichnet, daß man ein 2-Halogenpyridin der Formel VI 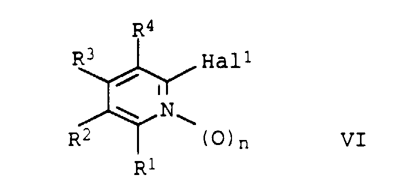 wobei R¹ bis R⁴ und der Index die in Anspruch 1 angegebenen Bedeutungen haben und Hal¹ für Chlor oder Brom steht, auf an sich bekannte Weise in einem inerten Lösungs- oder Verdünnungsmittel, in Gegenwart einer Base und eines Palladium- oder Nickel-Katalysators, mit einer Boronsäure der Formel VII 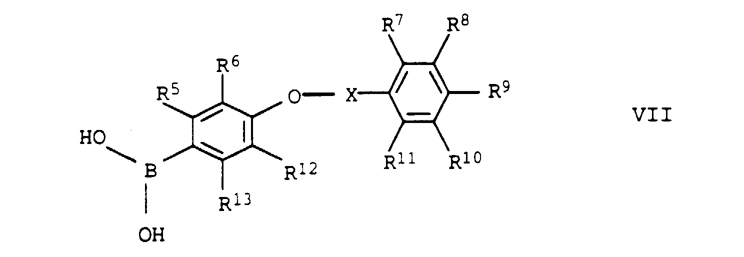 der einem Boroxin der Formel VIII 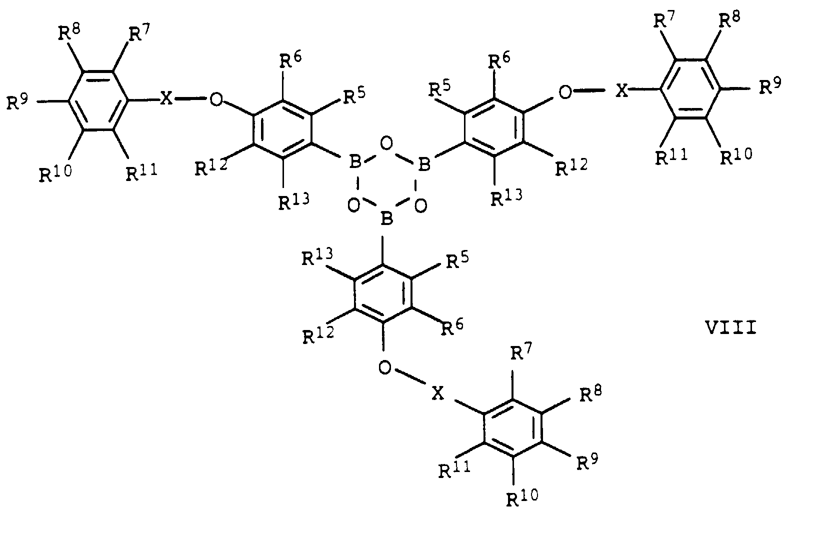 oder, sofern die Substituenten R¹ bis R¹³ unter den Bedingungen der Grignard-Reaktion inert sind, mit einer Grignard-Verbindung der Formel IX 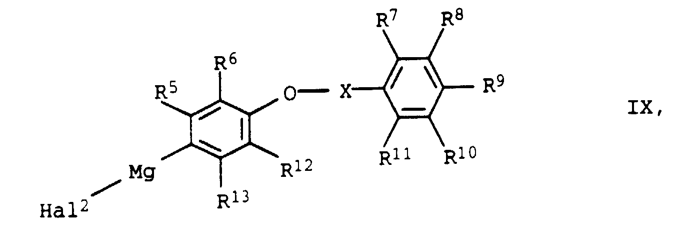 wobei jeweils X und R⁵ bis R¹³ die in Anspruch 1 angegebenen Bedeutungen haben und Hal² für Brom oder Jod steht, umsetzt.

## Claims

1. A substituted 2-phenylpyridine of the general formula I 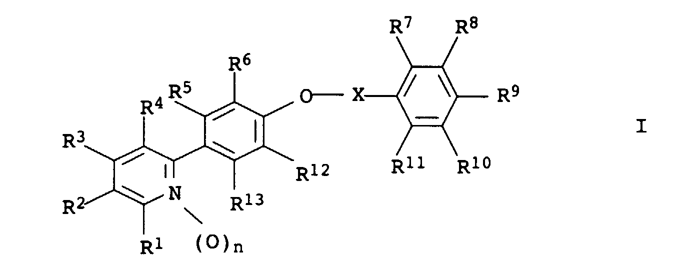 where the variables have the following meanings:
n is 0 or 1;
R¹, R³, R⁴ independently of one another are hydrogen, halogen, C₁-C₄-alkyl, C₁-C₄-haloalkyl, hydroxyl, C₁-C₄-alkoxy, C₁-C₄-haloalkoxy, nitro, amino, C₁-C₄-alkylamino, di(C₁-C₄-alkyl)amino, mercapto, C₁-C₄-alkylthio, C₁-C₄-haloalkylthio, cyano, hydroxycarbonyl, (C₁-C₄-alkoxy)carbonyl, aminocarbonyl, C₁-C₄-alkylaminocarbonyl or di(C₁-C₄-alkyl)aminocarbonyl;
R² is hydrogen, halogen, C₁-C₄-alkyl, C₁-C₄-haloalkyl, cyano, nitro, amino, hydroxyl, C₁-C₄-haloalkoxy, mercapto, C₁-C₄-alkylthio, C₁-C₄-haloalkylthio, C₁-C₄-alkylsulfinyl, C₁-C₄-haloalkylsulfinyl, C₁-C₄-alkylsulfonyl or C₁-C₄-haloalkylsulfonyl;
R⁵ is hydrogen, halogen or cyano;
R⁶ and R¹³ independently of one another are hydrogen or halogen;
X is methylene or carbonyl;
R⁷, R⁸, R⁹, R¹⁰ independently of one another are hydrogen, nitro, cyano, halogen, C₁-C₄-alkyl, C₁-C₄-haloalkyl, C₁-C₄-alkoxy or C₁-C₄-haloalkoxy;
R¹¹ is hydrogen, halogen, C₁-C₄-alkyl, C₁-C₄-haloalkyl, C₁-C₄-hydroxyalkyl, C₁-C₄-alkoxy-C₁-C₄-alkyl, C₃-C₆-cycloalkyl, C₂-C₄-alkenyl, C₂-C₄-alkynyl, hydroxycarbonyl-C₁-C₄-alkyl, hydroxycarbonyl-C₂-C₄-alkenyl, hydroxycarbonyl-C₂-C₄-alkynyl, (C₁-C₄-alkoxy)-carbonyl-C₁-C₄-alkyl, (C₁-C₄-alkoxy)-carbonyl-C₂-C₄-alkenyl, (C₁-C₄-alkoxy)-carbonyl-C₂-C₄-alkynyl, (C₁-C₄-alkoxy)-carbonyl-C₂-C₄-haloalkyl, (C₁-C₄-alkoxy)-carbonyl-C₂-C₄-haloalkenyl, aminocarbonyl-C₁-C₄-alkyl, aminocarbonyl-C₂-C₄-alkenyl, aminocarbonyl-C₂-C₄-alkynyl, C₁-C₄-alkylaminocarbonyl-C₁-C₄-alkyl, C₁-C₄-alkylaminocarbonyl-C₂-C₄-alkenyl, C₁-C₄-alkylaminocarbonyl-C₂-C₄-alkynyl, di(C₁-C₄-alkyl)aminocarbonyl-C₁-C₄-alkyl, di(C₁-C₄-alkyl)aminocarbonyl-C₂-C₄-alkenyl, di(C₁-C₄-alkyl)aminocarbonyl-C₂-C₄-alkynyl, aminocarbonyl-C₁-C₄-haloalkyl, aminocarbonyl-C₂-C₄-haloalkenyl, C₁-C₄-alkylaminocarbonyl-C₁-C₄-haloalkyl, C₁-C₄-alkylaminocarbonyl-C₂-C₄-haloalkenyl, di(C₁-C₄-alkyl)aminocarbonyl-C₂-C₄-haloalkyl, di(C₁-C₄-alkyl)aminocarbonyl-C₂-C₄-haloalkenyl, (hydroxycarbonyl-C₁-C₄-alkyl)aminocarbonyl-C₂-C₄-alkenyl, (hydroxycarbonyl-C₁-C₄-alkyl)aminocarbonyl-C₂-C₄-haloalkenyl, N-(hydroxycarbonyl-C₁-C₄-alkyl)-N-(C₁-C₄-alkyl)-aminocarbonyl-C₂-C₄-alkenyl, N-(hydroxycarbonyl-C₁-C₄-alkyl)-N-(C₁-C₄-alkyl)aminocarbonyl-C₂-C₄-haloalkenyl, [(C₁-C₄-alkoxy)carbonyl-C₁-C₄-alkyl]aminocarbonyl-C₂-C₄-alkenyl, [(C₁-C₄-alkoxy)carbonyl-C₁-C₄-alkyl]aminocarbonyl-C₂-C₄-haloalkenyl, N-[(C₁-C₄-alkoxy)carbonyl-C₁-C₄-alkyl]-N-(C₁-C₄-alkyl)-aminocarbonyl-C₂-C₄-alkenyl, N-[(C₁-C₄-alkoxy)carbonyl-C₁-C₄-alkyl]-N-(C₁-C₄-alkyl)aminocarbonyl-C₂-C₄-haloalkenyl, 2-[(C₁-C₄-alkoxy)carbonyl]pyrrolidin-1-yl-carbonyl-C₂-C₄-alkenyl, 2-[(C₁-C₄-alkoxy)carbonyl]-pyrrolidin-1-ylcarbonyl-C₂-C₄-haloalkenyl, formyl, (C₁-C₄-alkyl)carbonyl, (C₁-C₄-haloalkyl)carbonyl, hydroxyimino-C₁-C₄-alkyl, C₁-C₄-alkoxyimino-C₁-C₄-alkyl, 1,1-di(C₁-C₄-alkoxy)-C₁-C₄-alkyl, 1,1-di(C₁-C₄-alkylthio)-C₁-C₄-alkyl,
nitro, hydroxylamino, amino, C₁-C₄-alkyiamino, C₃-C₆-cycloalkylamino, di(C₁-C₄-alkyl)amino, (C₁-C₄-alkyl)carbonylamino, (C₁-C₄-haloalkyl)carbonylamino, C₁-C₄-alkylsulfonylamino, di(C₁-C₄-alkylsulfonyl)amino, C₁-C₄-haloalkylsulfonylamino, hydroxyl, C₁-C₄-alkoxy, C₁-C₄-haloalkoxy, cyano-C₁-C₄-alkoxy, C₁-C₄-alkoxy-C₁-C₄-alkoxy, C₁-C₄-alkylthio-C₁-C₄-alkoxy, C₃-C₆-cycloalkoxy, C₂-C₄-alkenyloxy, C₂-C₄-alkynyloxy, hydroxycarbonyl-C₁-C₄-alkoxy, (C₁-C₆-alkoxy)-carbonyl-C₁-C₄-alkoxy, C₁-C₄-alkoxy-(C₁-C₄-alkoxy)-carbonyl-C₁-C₄-alkoxy, (C₁-C₄-alkoxy)-carbonyl-(C₁-C₄-alkoxy)carbonyl-C₁-C₄-alkoxy, aminocarbonyl-C₁-C₄-alkoxy, C₁-C₄-alkylaminocarbonyl-C₁-C₄-alkoxy, di(C₁-C₄-alkyl)aminocarbonyl-C₁-C₄-alkoxy, l-pyrrolidinylcarbonyl-C₁-C₄-alkoxy, 1-piperidinylcarbonyl-C₁-C₄-alkoxy, 4-morpholinylcarbonyl-C₁-C₄-alkoxy, aminocarbonyl-(C₁-C₄-alkoxy)-carbonyl-C₁-C₄-alkoxy, (C₁-C₄-alkyl)aminocarbonyl-(C₁-C₄-alkoxy)carbonyl-C₁-C₄-alkoxy, di(C₁-C₄-alkyl)-aminocarbonyl-(C₁-C₄-alkoxy)carbonyl-C₁-C₄-alkoxy, 1-pyrrolidinylcarbonyl-(C₁-C₄-alkoxy)carbonyl-C₁-C₄-alkoxy, 1-piperidinylcarbonyl-(C₁-C₄-alkoxy)-carbonyl-C₁-C₄-alkoxy, 4-morpholinylcarbonyl-(C₁-C₄-alkoxy)carbonyl-C₁-C₄-alkoxy, [(C₁-C₄-alkoxy)-carbonyl-C₁-C₄-alkyl]aminocarbonyl-C₁-C₄-alkoxy, N-[(C₁-C₄-alkoxy)carbonyl-C₁-C₄-alkyl]-N-(C₁-C₄-alkyl)-aminocarbonyl-C₁-C₄-alkoxy, [2-[(C₁-C₄-alkoxy)-carbonyl]pyrrolidin-1-yl]carbonyl-C₁-C₄-alkoxy, cyano, hydroxycarbonyl, COCl, (C₁-C₄-alkoxy)carbonyl, (C₁-C₄-haloalkoxy)carbonyl, C₁-C₄-alkoxy-(C₁-C₄-alkoxy)-carbonyl, aminocarbonyl, C₁-C₄-alkylaminocarbonyl, di(C₁-C₄-alkyl)aminocarbonyl, C₃-C₆-cycloalkylaminocarbonyl, 1-pyrrolidinylcarbonyl, 1-piperidinyl-carbonyl, 4-morpholinylcarbonyl, hydroxycarbonyl-C₁-C₄-alkylaminocarbonyl, N-(hydroxycarbonyl-C₁-C₄-alkyl)-N-(C₁-C₄-alkyl)aminocarbonyl, (C₁-C₄-alkoxy)carbonyl-C₁-C₄-alkylaminocarbonyl, N-[(C₁-C₄-alkoxy)carbonyl-C₁-C₄-alkyl]-N-(C₁-C₄-alkyl)-aminocarbonyl, 2-hydroxycarbonylpyrrolidin-1-yl-carbonyl, 2-[(C₁-C₄-alkoxy)carbonyl]pyrrolidin-1-yl-carbonyl or a group 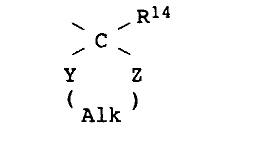 where
Y and Z independently of one another are oxygen or sulfur,
R¹⁴ is hydrogen, C₁-C₄-alkyl or (C₁-C₄-alkoxy)carbonyl and
Alk is an ethylene or trimethylene chain where one or more hydrogen atoms can be replaced in each case by C₁-C₄-alkyl or (C₁-C₄-alkoxy)carbonyl;
R¹² is hydrogen, nitro, amino, hydroxylamino, C₁-C₄-alkylsulfonylamino, di(C₁-C₄-alkylsulfonyl)amino, C₁-C₄-haloalkylsulfonylamino, (C₁-C₄-alkoxy)carbonyl-C₁-C₄-haloalkyl or (C₁-C₄-alkoxy)carbonyl-C₂-C₄-haloalkenyl;
with the proviso that R⁵, R⁷, R¹¹ and R¹³ are not all simultaneously hydrogen or R⁷ and R⁸ or R¹⁰ and R¹¹ are not simultaneously fluorine,
or, if applicable, an agriculturally useful salt of a compound I.

2. A substituted 2-phenylpyridine of the formula I as claimed in claim 1 where R¹ and R³ are hydrogen, R² is C₁-C₄-haloalkyl and R⁴ is halogen.

3. The use of substituted 2-phenylpyridines of the formula I' or of an agriculturally useful salt thereof, where I' corresponds to the definition of I as claimed in claim 1, but without the proviso, as herbicides or for desiccating and/or defoliating plants.

4. A herbicidal composition which comprises a herbicidally active amount of at least one substituted 2-phenylpyridine of the formula I or of an agriculturally useful salt of I, and at least one inert liquid and/or solid carrier and, if desired, at least one surfactant.

5. A composition for desiccating and/or defoliating plants which comprises such an amount of at least one substituted 2-phenylpyridine of the formula I or of an agriculturally useful salt of I that it acts as a desiccant and/or defoliant, and at least one inert liquid and/or solid carrier and, if desired, at least one surfactant.

6. A process for the preparation of herbicidally active compositions, which comprises mixing a herbicidally active amount of at least one substituted 2-phenylpyridine of the formula I or of an agriculturally useful salt of I, and of at least one inert liquid and/or solid carrier and, if desired, at least one surfactant.

7. A process for the preparation of compositions which act as desiccants and/or defoliants, which comprises mixing such an amount of at least one substituted 2-phenylpyridine of the formula I or of an agriculaturally useful salt of I that it acts as a desiccant and/or defoliant, and at least one inert liquid and/or solid carrier and, if desired, at least one surfactant.

8. A method of controlling undesirable vegetation, which comprises allowing a herbicidally active amount of at least one substituted 2-phenylpyridine of the formula I' or of an agriculturally useful salt of I', where I' corresponds to the definition of I as claimed in claim 1 but without the proviso, to act on plants, their environment or seeds.

9. A method of desiccating and/or defoliating plants, which comprises allowing an amount having a desiccant and/or defoliant action of at least one substituted 2-phenylpyridine of the formula I' or of an agriculturally useful salt of I', where I' corresponds to the definition of I as claimed in claim 1 but without the proviso, to act on plants.

10. A method as claimed in claim 9, wherein cotton is treated.

11. A process for the preparation of substituted
2-phenylpyridines of the formula I as claimed in claim 1 where X is methylene, which comprises reacting a 2-(4-hydroxyphenyl)pyridine of the formula II 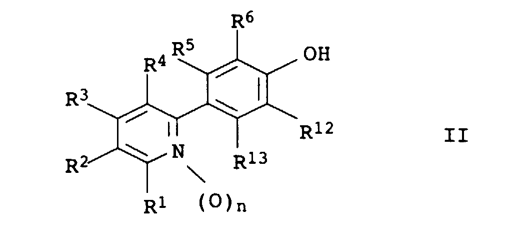 where the variables have the meanings specified in claim 1 with a benzyl halide or benzyl mesylate of the formula III 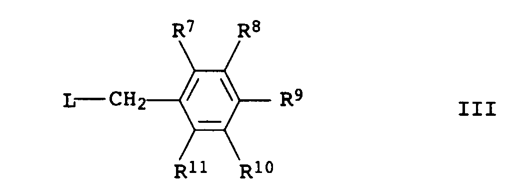 where L is chlorine, bromine or methylsulfonyloxy in a manner known per se in an inert solvent or diluent, if desired in the presence of a base.

12. A process for the preparation of substituted 2-phenylpyridines of the formula I as claimed in claim 1 where X is carbonyl, which comprises reacting a 2-(4-hydroxyphenyl)pyridine of the formula II 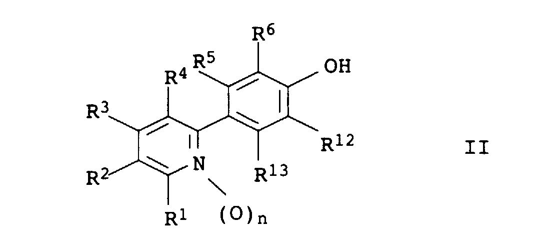 where the variables have the meanings specified in claim 1 with a benzoyl halide of the formula IV 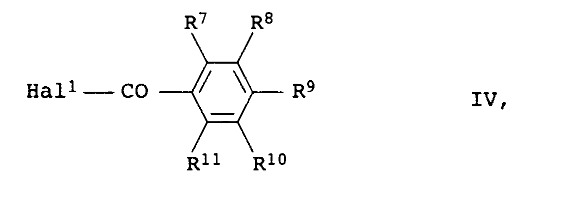 where Hal¹ is chlorine or bromine in a manner known per se in an inert solvent or diluent, if desired in the presence of a base.

13. A process for the preparation of substituted 2-phenylpyridines of the formula I as claimed in claim 1 where X is carbonyl and R¹¹ is hydroxycarbonyl, which comprises reacting a 2-(4-hydroxyphenyl)pyridine of the formula II 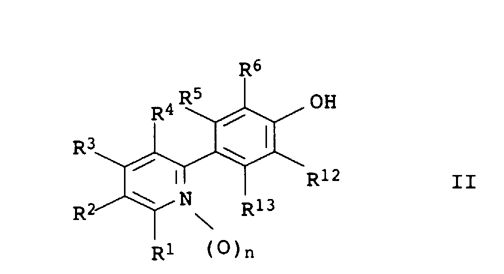 where the variables have the meanings specified in claim 1 with a substituted phthalic anhydride of the formula V 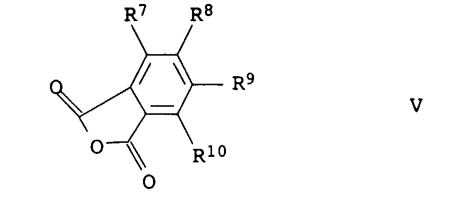 in a manner known per se in an inert solvent or diluent.

14. A process for the preparation of substituted 2-phenylpyridines of the formula I as claimed in claim 1 where n is 1, which comprises oxidizing the corresponding substituted 2-phenylpyridine in which n is 0 in a manner known per se.

15. The use of the 2-(4-hydroxyphenyl)pyridines of the formula II where the variables have the meanings specified in claim 1 for the preparation of substituted 2-phenylpyridines of the formula I as claimed in claim 1.

16. A process for the preparation of substituted 2-phenylpyridines of the formula I as claimed in claim 1, which comprises reacting a 2-halopyridine of the formula VI 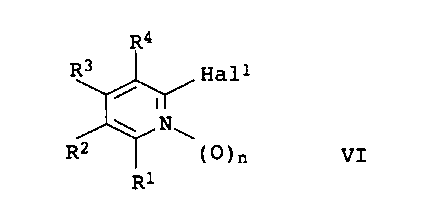 where R¹ to R⁴ and the index have the meanings specified in claim 1 and Hal¹ is chlorine or bromine with a boronic acid of the formula VII 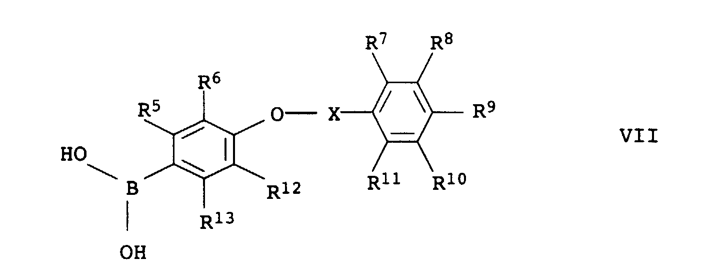 or with a boroxine of the formula VIII 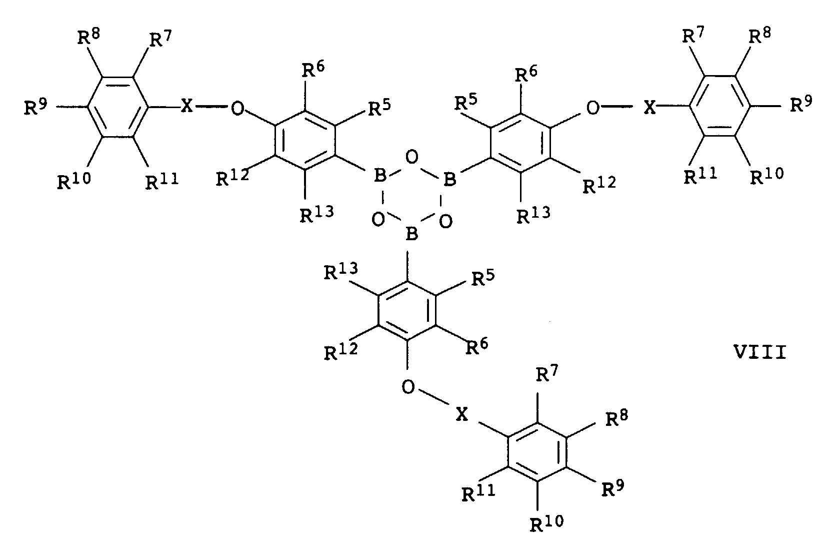 or, if the substituents R¹ to R¹³ are inert under the conditions of a Grignard reaction, with a Grignard compound of the formula IX 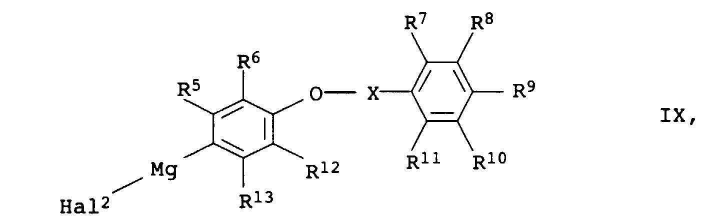 where X and R⁵ to R¹³ in each case have the meanings specified in claim 1 and Hal² is bromine or iodine,
in a manner known per se in an inert solvent or diluent in the presence of a base and of a palladium or nickel catalyst.

## Revendications

1. 2-phénylpyridines substituées de formule générale I 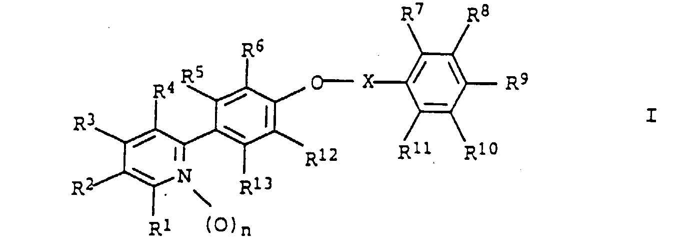 dans laquelle les symboles ont les significations suivantes :
n est égal à 0 ou 1 ;
R¹, R³, R⁴ représentent chacun, indépendamment les uns des autres, l'hydrogène, un halogène, un groupe alkyle en C1-C4, halogénoalkyle en C1-C4, hydroxy, alcoxy en C1-C4, halogénoalcoxy en C1-C4, nitro, amino, alkylamino en C1-C4, di-(alkyle en C1-C4)amino, mercapto, alkylthio en C1-C4, halogénoalkylthio en C1-C4, cyano, hydroxycarbonyle, (alcoxy en C1-C4)carbonyle, aminocarbonyle, (alkyle en C1-C4)aminocarbonyle ou di-(alkyle en C1-C4)aminocarbonyle ;
R² représente l'hydrogène, un halogène, un groupe alkyle en C1-C4, halogénoalkyle en C1-C4, cyano, nitro, amino, hydroxy, halogénoalcoxy en C1-C4, mercapto, alkylthio en C1-C4, halogénoalkylthio en C1-C4, alkylsulfinyle en C1-C4, halogénoalkylsulfinyle en C1-C4, alkylsulfonyle en C1-C4 ou halogénoalkylsulfonyle en C1-C4;
R⁵ représente l'hydrogène, un halogène ou un groupe cyano ;
R⁶ et R¹³ représentent chacun, indépendamment l'un de l'autre, l'hydrogène ou un halogène ;
X représente un groupe méthylène ou carbonyle ;
R⁷, R⁸, R⁹, R¹⁰ représentent chacun, indépendamment les uns des autres,
l'hydrogène, un groupe nitro, cyano, un halogène, un groupe alkyle en C1-C4, halogéno-alkyle en C1-C4, alcoxy en C1-C4 ou halogénoalcoxy en C1-C4;
R¹¹ représente l'hydrogène, un halogène, un groupe alkyle en C1-C4, halogénoalkyle en C1-C4, hydroxyalkyle en C1-C4, (alcoxy en C1-C4)alkyle en C1-C4, cycloalkyle en C3-C6, alcényle en C2-C4, alcynyle en C2-C4, hydroxycarbonyl-alkyle en C1-C4, hydroxycarbonyl-alcényle en C2-C4, hydroxycarbonyl-alcynyle en C2-C4, (alcoxy en C1-C4)carbonyl-alkyle en C1-C4, (alcoxy en C1-C4)carbonyl-alcényle en C2-C4, (alcoxy en C1-C4)carbonyle-alcynyle en C2-C4, (alcoxy en C1-C4)carbonyl-halogénoalkyle en C2-C4, (alcoxy en C1-C4)carbonyl-halogénoalcényle en C2-C4, aminocarbonylalkyle en C1-C4, aminocarbonyl-alcényle en C2-C4, aminocarbonyl-alcynyle en C2-C4, (alkyle en C1-C4)aminocarbonyl-alkyle en C1-C4, (alkyle en C1-C4)aminocarbonyl-alcényle en C2-C4, (alkyle en C1-C4)aminocarbonyl-alcynyle en C2-C4, di-(alkyle en C1-C4)aminocarbonyl-alkyle en C1-C4, di-(alkyle en C1-C4)amino-carbonyl-alcényle en C2-C4, di-(alkyle en C1-C4)aminocarbonyl-alcynyle en C2-C4, aminocarbonyl-halogénoalkyle en C1-C4, aminocarbonyl-halogénoalcényle en C2-C4, (alkyle en C1-C4)aminocarbonyl-halogénoalkyle en C1-C4, (alkyle en C1-C4)aminocarbonyl-halogénoalcényle en C2-C4, di-(alkyle en C1-C4)aminocarbonyl-halogénoalkyle en C2-C4, di-(alkyle en C1-C4)aminocarbonyl-halogénoalcényle en C2-C4, (hydroxycarbonyl-alkyle en C1-C4)aminocarbonyl-alcényle en C2-C4, (hydroxycarbonyl-alkyle en C1-C4)aminocarbonyl-halogénoalcényle en C2-C4, N-(hydroxycarbonyl-alkyle en C1-C4)-N-(alkyle en C1-C4)-aminocarbonyl-alcényle en C2-C4, N-(hydroxycarbonyl-alkyle en C1-C4)-N-(alkyle en C1-C4)-aminocarbonyl-halogénoalcényle en C2-C4, [(alcoxy en C1-C4)carbonyl-alkyle en C1-C4] - aminocarbonyl-alcényle en C2-C4, [(alcoxy en C1-C4)carbonyl-alkyle en C1-C4] aminocarbonyl-halogénoalcényle en C2-C4, N- (alcoxy en C1-C4)carbonyl-alkyle en C1-C4 -N-(alkyle en C1-C4)aminocarbonyl-alcényle en C2-C4, N- [(alcoxy en C1-C4)carbonyl-alkyle en C1-C4)-N-(alkyle en C1-C4)aminocarbonyl-halogénoalcényle en C2-C4, 2-[(alcoxy en C1-C4)carbonyl] - pyrrolidin-1-yl-carbonyl-alcényle en C2-C4, 2-[(alcoxy en C1-C₄)carbony1]pyrrolidin-1-yl-carbonyl-halogénoalcényle en C2-C4, formyle, (alkyle en C1-C4)carbonyle, (halogéno-alkyle en C1-C4)carbonyle, hydroxyimino-alkyle en C1-C4, (alcoxy en C1-C4)imino-alkyle en C1-C4, 1,1-di-(alcoxy en C1-C4)alkyle en C1-C4, 1,1-di-(alkylthio en C1-C4)alkyle en C1-C4, nitro, hydroxylamino, amino, alkylamino en C1-C4, cycloalkylamino en C3-C6, di-(alkyle en C1-C4)amino, (alkyle en C1-C4)carbonylamino, (halogénoalkyle en C1-C4)carbonylamino, alkylsulfonylamino en C1-C4, di-(alkylsulfonyle en C1-C4)amino, halogénoalkylsulfonylamino en C1-C4, hydroxy, alcoxy en C1-C4, halogénoalcoxy en C1-C4 cyano-alcoxy en C1-C4, (alcoxy en C1-C4)alcoxy en C1-C4, (alkylthio en C1-C4)alcoxy en C1-C4, cycloalcoxy en C3-C6, alcényloxy en C2-C4, alcinyloxy en C2-C4, hydroxycarbonyl-alcoxy en C1-C4, (alcoxy en C1-C6)carbonyl-alcoxy en C1-C4, (alcoxy en C1-C4)-(alcoxy en C1-C4)carbonyl-alcoxy en C1-C4, (alcoxy en C1-C4)carbonyl-(alcoxy en C1-C4)carbonyl-alcoxy en C1-C4, aminocarbonyl-alcoxy en C1-C4, (alkyle en C1-C4)aminocarbonyl-alcoxy en C1-C4, di-(alkyle en C1-C4)aminocarbonyl-alcoxy en C1-C4, 1-pyrrolidinyl-carbonyl-alcoxy en C1-C4, 1-pipéridinyl-carbonyl-alcoxy en C1-C4, 4-morpholinyl-carbonyl-alcoxy en C1-C4, amino-carbonyl-(alcoxy en C1-C4)carbonyl-alcoxy en C1-C4, (alkyle en C1-C4)aminocarbonyl-(alcoxy en C1-C4) carbonyl-(alcoxy en C1-C4), di-(alkyle en C1-C4)aminocarbonyl-(alcoxy en C1-C4)carbonyl-alcoxy en C1-C4, 1-pyrrolidinyl-carbonyl-(alcoxy en C1-C4)carbonyl-alcoxy en C1-C4, 1 -pipéridinylcarbonyl-(alcoxy en C1-C4)carbonyl-alcoxy en C1-C4, 4-morpholinyl-carbonyl-(alcoxy en C1-C4)carbonyl-alcoxy en C1-C4 [(alcoxy en C1-C4)-carbonyl-alkyle en C1-C4] aminocarbonyl-alcoxy en C1-C4, N-[(alcoxy en C1-C4)carbonyl-alkyle en C1-C4]-N-(alkyle en C1-C4)aminocarbonyl-alcoxy en C1-C4, [2- [(alcoxy en C1-C4)-carbonyl]-pyrrolidin-1-yl] -carbonyl-alcoxy en C1-C4, cyano, hydroxycarbonyle, COC1, (alcoxy en C1-C4)carbonyle, (halogénoalcoxy en C1-C4)carbonyle, alcoxy en C1-C4-(alcoxy en C1-C4)carbonyle, aminocarbonyle, (alkyle en C1-C4)aminocarbonyle, di-(alkyle en C1-C4)aminocarbonyle, (cycloalkyle en C3-C6)aminocarbonyle, 1-pyrrolidinylcarbonyle, 1-pipéridinylcarbonyle, 4-morpholinylcarbonyle, hydroxycarbonyl-(alkyle en C1-C4)amine-carbonyle, N-(hydroxycarbonyl-alkyle en C1-C4)-N-(alkyle en C1-C4)aminocarbonyle, (alcoxy en C1-C4)carbonyl-(alkyle en C1-C4)aminocarbonyle, N-[(alcoxy en C1-C4)carbonyl-alkyle en C1-C4]-N-(alkyle en C1-C4)aminocarbonyle, 2-hydroxycarbonyl-pyrrolidin-1-yl-carbonyle, 2-[(alcoxy en C1-C4)carbonyl] pyrrolidin-1-yl-carbonyle ou bien un groupe 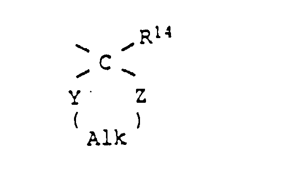
dans lequel,
Y et Z représentent chacun, indépendamment l'un de l'autre, l'oxygène ou le soufre,
R¹⁴ représente l'hydrogène, un groupe alkyle en C1-C4 ou (alcoxy en C1-C4) carbonyle et
Alk représente une chaîne éthylène ou triméthylène dans laquelle un ou plusieurs atomes d'hydrogène peuvent être remplacés chacun par un groupe alkyle en C1-C4 ou (alcoxy en C1-C4)carbonyle ;
R¹² représente l'hydrogène, un groupe nitro, amino, hydroxylamino, alkylsulfonylamino en C1-C4, di-(alkylsulfonyle en C1-C4)amino, halogénoalkylsulfonylamino en C1-C4, (alcoxy en C1-C4)carbonyl-halogénoalkyle en C1-C4 ou (alcoxy en C1-C4)carbonyl-halogénoalcényle en C2-C4,
sous réserve que R⁵, R⁷, R¹¹ et R¹³ ne peuvent pas tous représenter simultanément l'hydrogène ou que R⁷ et R⁸ ou R¹⁰ et R¹¹ ne peuvent représenter simultanément le fluor, ainsi que les sels des composés I convenant pour les applications agricoles autant qu'ils existent.

2. 2-phénylpyridines substituées de formule I selon la revendication 1, dans laquelle R¹ et R³ représentent l'hydrogène, R² un groupe halogénoalkyle en C1-C4 et R⁴ un halogène.

3. Utilisation des 2-phénylpyridines substituées de formule I' et de leurs sels acceptables pour les usages agricoles, I' correspondant à I de la revendication 1 mais sans la réserve, en tant qu'herbicides ou pour la dessication et/ou la /défoliation de végétaux.

4. Produit herbicide contenant une quantité herbicide efficace d'au moins une 2-phénylpyridine substituée de formule I ou de l'un de ses sels et au moins un véhicule liquide et/ou solide inerte avec, si on le désire, au moins une substance tensio-active.

5. Produit pour la dessication et/ou la défoliation des végétaux, contenant une quantité dessiccante et/ou défoliante efficace d'au moins une 2-phénylpyridine substituée de formule I ou de l'un de ses sels, et au moins un véhicule liquide et/ou solide inerte avec, si on le désire, au moins une substance tensio-active.

6. Procédé pour préparer des produits herbicides, caractérisé par le fait que l'on mélange une quantité herbicide efficace d'au moins 2-phénylpyridine substituée de formule I ou de l'un de ses sels acceptables pour les applications agricoles, et au moins un véhicule liquide et/ou solide inerte ainsi que, si on le désire, au moins une substance tensio-active.

7. Procédé pour la préparation de produits dessicants et/ou défoliants, caractérisé par le fait que l'on mélange une quantité dessicante et/ou défoliante efficace d'au moins une 2-phénylpyridine substituée de formule I ou de l'un de ses sels acceptables pour les applications agricoles, et au moins un véhicule liquide et/ou solide inerte ainsi que, si on le désire, au moins une substance tensio-active.

8. Procédé pour combattre les croissances de végétaux indésirables, caractérisé par le fait que l'on fait agir une quantité herbicide efficace d'au moins une 2-phénylpyridine substituée de formule I' ou de l'un de ses sels acceptables pour les usages agricoles, I' correspondant à I de la revendication 1 mais sans la réserve, sur les végétaux, leur habitat ou sur les semences.

9. Procédé pour la dessication et/ou la défoliation de végétaux, caractérisé par le fait que l'on fait agir une quantité dessicante et/ou défoliante efficace d'au moins une 2-phénylpyridine substituée de formule I' ou de l'un de ses sels acceptables pour les usages agricoles, I' correspondant à I de la revendication 1 mais sans la réserve, sur les végétaux.

10. Procédé selon la revendication 9, caractérisé par le fait que l'on traite le coton.

11. Procédé de préparation des 2-phénylpyridines substituées de formule I de la revendication 1 dans laquelle X représente le groupe méthylène, caractérisé par le fait que l'on fait réagir une 2-(4-hydroxyphényl)pyridine de formule II 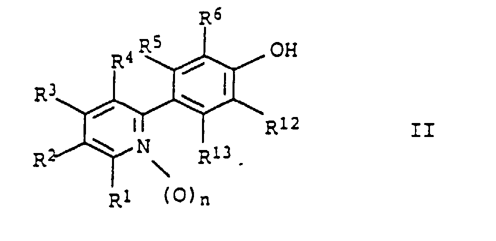 dans laquelle les symboles ont les significations indiquées dans la revendication 1, de manière connue en soi, dans un solvant ou diluant inerte et le cas échéant en présence d'une base, avec un halogénure ou un méthanesulfonate de benzyle de formule III 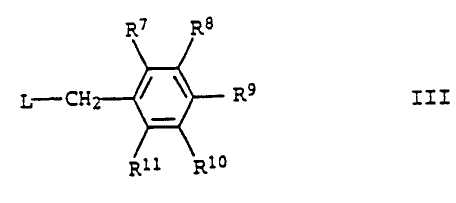 dans laquelle L représente le chlore, le brome ou le groupe méthylsulfonyloxy.

12. Procédé de préparation des 2-phénylpyridines substituées de formule I de la revendication 1 dans laquelle X représente le groupe carbonyle, caractérisé par le fait que l'on fait réagir une 2-(4-hydroxyphényl)pyridine de formule II 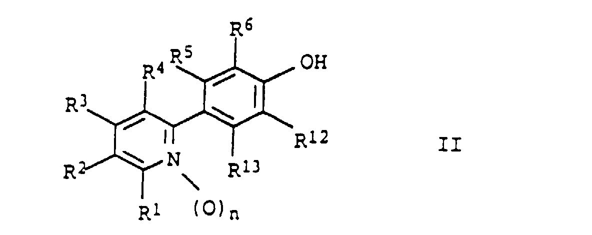 dans laquelle les symboles ont les significations indiquées dans la revendication 1, de manière connue en soi, dans un solvant où diluant inerte, le cas échéant en présence d'une base, avec un halogénure de benzoyle de formule IV 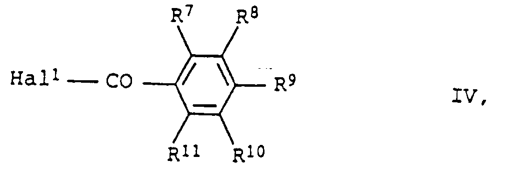 dans laquelle Hal¹représente le chlore ou le brome.

13. Procédé de préparation des 2-phénylpyridines substituées de formule I de la revendication 1 dans laquelle X représente un groupe carbonyle et R¹¹ un groupe hydroxy-carbonyle, caractérisé par le fait que l'on fait réagir une 2-(4-hydroxyphényl)pyridine de formule II 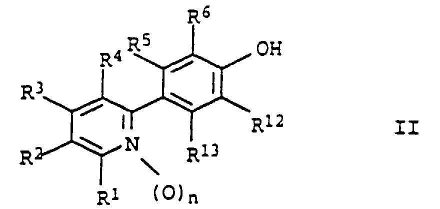 dans laquelle les symboles ont les significations indiquées dans la revendication 1, de manière connue en soi, dans un solvant ou diluant inerte, avec un anhydride phtalique substitué de formule V

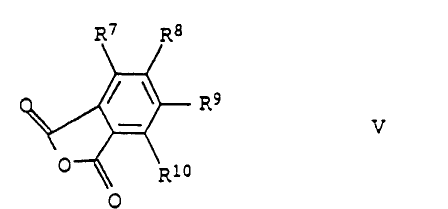

14. Procédé de préparation des 2-phénylpyridines substituées de formule I de la revendication 1 dans laquelle n est égal à 1, caractérisé par le fait que l'on oxyde de manière connue en soi la 2-phénylpyridine substituée correspondante pour laquelle n est égal à 0.

15. Utilisation des 2-(4-hydroxyphényl)pyridines de formule II dans laquelle les symboles ont les significations indiquées dans la revendication 1, pour la préparation des 2-phénylpyridines substituées de formule I de la revendication 1.

16. Procédé de préparation des 2-phénylpyridines substituées de formule I de la revendication 1, caractérisé par le fait que l'on fait réagir une 2-halogénopyridine de formule VI 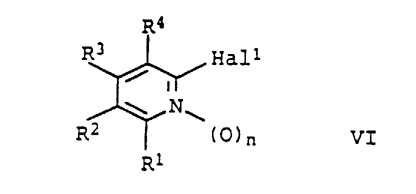 dans laquelle R¹ à R⁴ et l'indice ont les significations indiquées dans la revendication 1, et Hal¹ représente le chlore ou le brome, de manière connue en soi, dans un solvant ou diluant inerte, en présence d'une base et d'un catalyseur au palladium ou au nickel, avec un acide boronique de formule VII 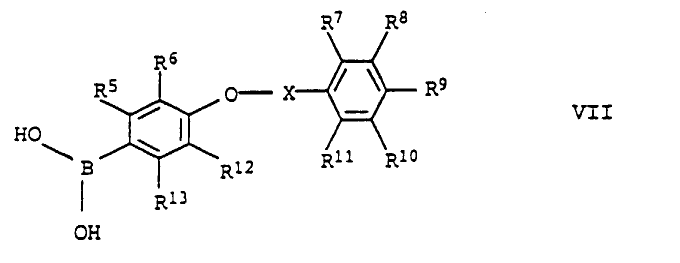 ou une boroxine de formule VIII 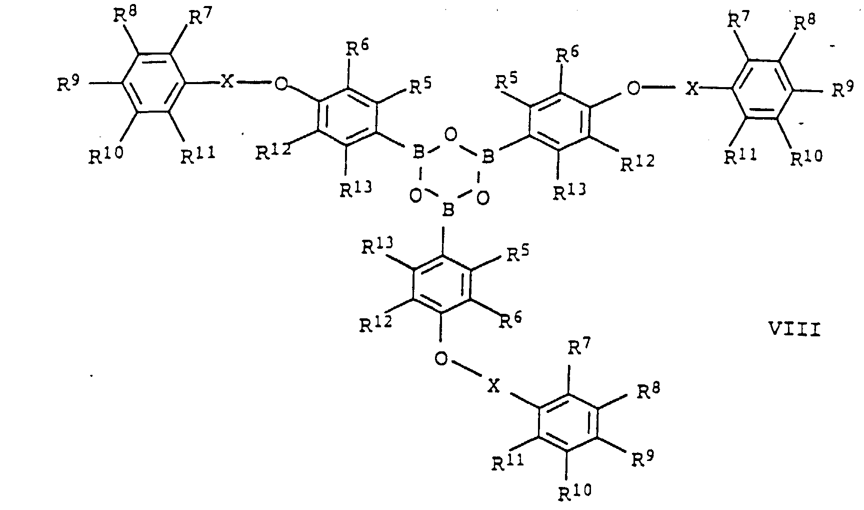 ou bien, pour autant que les substituants R¹ à R¹³ soient inertes dans les conditions de la réaction de Grignard, avec un dérivé de Grignard répondant à la formule IX 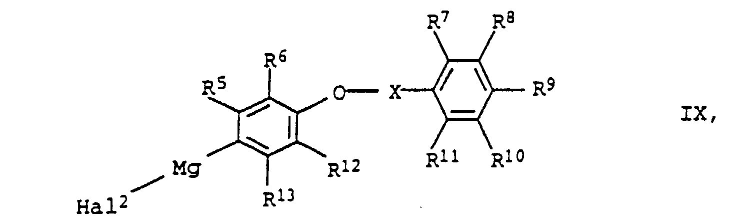 dans laquelle X et R⁵ à R¹³ ont les significations indiquées dans la revendication 1 et Hal² représente le brome ou l'iode.
